# EUROPEAN PATENT APPLICATION

(11) **EP 2 014 768 A1**
(43) Date of publication of application: **14.01.2009**
(21) Application number: 07013489.5
(22) Date of filing: 10.07.2007
(51) Int. Cl.: C12N 5/06

(54) **Neurons and methods for preparing them**

(71) Applicant: Innovalor AG, 6442 Gersau (CH)
(72) Inventor: Poppe, Monika, 8106 Adlikon (CH); Schmiedeknecht, Anett, 04229 Leipzig (DE); Peschel, Horst, 51643 Gummersbach (DE)
(74) Representative: Schalch, Rainer

(57) **Abstract**

The present invention provides an improved method for generating mature neurons and dopaminergic neurons from neural precursor cells (NPCs). The cells obtained by the method of the invention are useful in the treatment of neurodegenerative disorders.

## Description

The present invention provides an improved method for culturing neural precursor cells (NPCs). The method allows the proliferation and/or the dopaminergic differentiation of the NPCs. The cells obtained by the method of the invention are useful in the treatment of neurodegenerative disorders.

Disorders of the central nervous system (CNS) encompass numerous afflictions such as neurodegenerative diseases, acute brain injury and a large number of CNS dysfunctions. In recent years neurodegenerative diseases have become an important concern due to the expanding elderly population which is at the greatest risk for these disorders. The diseases which include for example Alzheimer's disease and Parkinson's disease are linked to the degeneration of neuronal cells in particular locations of the CNS leading to the inability of these cells, or the brain region, to carry out their intended function.

Unfortunately, the ability of the adult functional nervous system to produce new neural cells is quite limited as neurogenesis in mammals is largely completed early in the postnatal period. This relative inability to produce new neural cells in most mammals (and especially in primates) may be advantageous for long-term memory retention, however it is a distinct disadvantage when the need to replace lost neuronal cells arises due to an injury or disease.

To date treatment for CNS disorders is primarily carried out via the administration of pharmaceutical compounds. Unfortunately this type of treatment has shown limited efficacy and many complications including limited ability to transport drugs across the blood-brain-barrier and drug tolerance acquired by patients to whom these drugs are administered long-term. For instance partial restoration of dopaminergic activity in Parkinson's patients has been achieved with "levodopa" (L-DOPA) which is a dopamine precursor able to cross the blood-brain-barrier. However, patients become tolerant to the effects of "levodopa" and therefore steadily increasing dosages are needed to maintain its effects. In addition there are a number of side effects associated with "levodopa" such as increased and uncontrollable movement.

Recently it was found that the concept of neurological tissue transplantation can be applied to the treatment of neurological diseases such as Parkinson's disease. Neural grafts may avert the need not only for constant drug administration but also for complicated drug delivery systems which arise due to the blood-brain-barrier. Especially NPCs promise treatment for currently incurable brain diseases and injuries. Based on their unique capacity to migrate throughout the brain and to target invading tumor cells, transplantation of NPCs offers a new potential therapeutic approach as a cell based delivery system for gene therapy in brain tumors (Shah et al., Neural precursor cells and their role in neuro-oncology, Dev Neurosci 26: 118-130; 2004).

However, there are limitations to this technique as well. Cells used for transplantation which carry cell surface molecules of a differentiated cell from another host can induce an immune reaction in the host. In addition cells must be at a stage of development in that they are able to form normal neural connections as neighbouring cells. For these reasons initial studies on neurotransplantation centered on the use of fetal (embryonic) cells: Several studies have shown improvements in patients with Parkinson's disease after receiving grafts of fetal CNS tissue.

Though those studies are encouraging, there are further problems associated with neurological tissue transplantation as - for example - the use of large quantities of aborted fetal tissue for the treatment of disease raises ethical considerations and political obstacles. In addition there are serious doubts as to whether sufficient supply of fetal tissue would be available for transplantation.

There are further considerations as well: It exists for example a potential of contamination during fetal tissue preparation. Moreover the tissue may already be infected with bacteria or virus and thus requires extensive diagnostic testing for each fetus used. However, even diagnostic testing might not uncover all infected tissue. For example the successful diagnoses of HIV-free tissue is not guaranteed because antibodies to the virus are generally not present until several weeks after infection.

There is also the added problem that fetal CNS tissue is composed of more than one cell type and thus is not a well defined source of tissue. Specific applications mostly require specifically differentiated cells; for example for the treatment of Parkinson's disease only dopaminergic cells are required. The ability to tightly control differentiation or form homogenous populations of partially differentiated or terminally differentiated cells by differentiation in vitro has been proved problematic. Uncontrolled differentiation induces mixtures of pluripotent stem cells and partially differentiated stem/precursor cells corresponding to various cell lineages. When these fetal-derived stem cell mixtures are transplantated into a recipient tissue, the contaminating pluripotent stem cells can proliferate and differentiate to form tumors while partially differentiated stem and precursor cells can further differentiate to form a mixture of inappropriate and undesired cell types. It is well known from studies in animal models that tumors originating from contaminating pluripotent cells can cause catastrophic tissue damage and death. In addition pluripotent cells contaminating a cell transplant can generate various inappropriate stem cells, precursor cells and differentiated cell types in the donor which can lead to the formation of inappropriate tissues within a cell transplant. These outcomes cannot be tolerated for clinical applications in humans.

Therefore, uncontrolled fetal-derived stem cell differentiation makes the clinical use of fetal-derived cells in human cell therapies impossible. While currently available transplantation approaches represent a significant improvement over other available treatments for neurological disorders, the transplantated cells - and therefore also the culture conditions - still need to be optimized before this transplantation method can be used clinically.

It has proven difficult to culture NPCs over longer periods of time and at the same time to retain the differentiation potential of the NPCs. The rate of proliferation of human NPCs is limited (Ostenfeld et al. (2000) Exp Neurol. 164(1):215-226). Thus, extended expansion and modification of such cells is limited. Using low oxygen conditions, the applicants have for the first time developed culture systems that allow for expansion of > 50 passages without affecting the potential of such precursor cells to differentiate into neurons or glia. Such long-term culture systems also allow for partial differentiation and modulation of such cells via long-term application of novel small molecules (see WO 00/78931 A2, filed by NeuroProgen GmbH). Therefore, the percentage of specific cells (e. g. dopaminergic neurons) can be markedly increased. However, there is still the need for further improved methods of culturing NPCs in order to obtain better differentiation of NPCs into neuronal cells, especially into dopaminergic neuronal cells. In particular, cells to be transplanted should be fully mature neurons, i.e. they should be capable of firing action potentials and express markers of mature neurons. There are many reports in the prior art on methods and protocols for differentiating cells into neuronal cells. However, while the cells obtained by these prior art methods are often termed "differentiated neuronal cells" or the like, they are still not fully mature neurons.

The inventors of the present application surprisingly found that subjecting neuronal cells or neural precursor cells to a hyperpolarization step greatly enhances the maturation of the cells. As a result, the thus treated neuronal cells are capable of firing action potentials. This effect was even more pronounced if the cells were subjected to a depolarization step prior to the hyperpolarization step. Partially differentiated cells were exposed to media that induce hyperpolarization of neuronal precursor cells. This final step of maturation is required to render cells fully mature, i. e. fire action potentials, express a variety of markers of mature neurons. It can be performed in the presence of small molecules (nucleotides, forskolin, etc.) growth factors or cytokines.

### SUMMARY OF INVENTION

In a first aspect, the present invention relates to a method for generating mature neuronal cells, comprising (a) expanding and/or differentiating neural precursor cells and (b) subjecting the cells obtained in step (a) to a hyperpolarization step. The neural precursor cells (NPCs) are preferably human NPCs.

In a second aspect, the present invention relates to a method for generating dopaminergic neurons, comprising exposing neural precursor cells to forskoline, interleukin 1, FGF-8, at least one uracil nucleotide, dexamethasone and at least one glycogen synthase kinase 3 (GSK-3) inhibitor such as kenpaullone or 6-bromoindirubin-3'-oxime (BIO). The method of the invention comprises (i) expanding a first population of cells comprising neural precursor cells by exposing the cells to at least one growth factor so as to obtain a second population of cells, and (ii) exposing the second population of cells to forskoline, interleukin 1, FGF-8, at least one uracil nucleotide, dexamethasone and at least one GSK-3 inhibitor (e.g. kenpaullone and/or BIO) to obtain a third population of cells comprising dopaminergic neuronal cells.

In a third aspect, the invention relates to a mature neuronal cell and to a population of cells obtainable by the method of the invention.

In a fourth aspect, the invention relates to a pharmaceutical composition comprising the population of cells according to the invention.

In a fifth aspect, the invention relates to the use of a population of cells according to the invention for the manufacture of a medicament for the treatment of a neurodegenerative disorder, in particular Parkinson's Disease.

### DETAILED DESCRIPTION OF THE INVENTION

### Definitions

A "Neural precursor cell" or "NPC", as used herein, is a cell that is capable of self-renewing and of differentiating into glial cells and neurons. Usually, non-differentiated NPCs express the marker molecules nestin, CD15, CD56, CD90, CD164, and NGFR, whereas they do not express CD45, CD105 (endoglin), CD109, and CD140b (PDGF-RB) [Vogel, W. et al. Heterogeneity among human bone marrow-derived mesenchymal stem cells and neural progenitor cells. J. Haematol. 88, 126-133 (2003); the disclosure of which is incorporated herein by reference]. The term "self-renewing" refers to the capability of a cell to undergo mitosis and to divide to produce two daughter cells.

Human midbrain derived NPCs further express the purinergic receptors P2Y₁, P2Y₄, P2Y₆, and P2X₄.

"Neuronal cells" are cells that express the marker protein microtubule-associated protein-2 (MAP-2). They may further express the marker proteins neurofilament, calbindin and tau. The terms "neurons" and "mature neuronal cells" as used herein denote post-mitotic neuronal cells that exhibit characteristic electrophysiological properties as described , hereinafter. These consist of typical fast sodium currents and large potassium currents following depolarization. In addition, one can measure ligand gated ion currents (e. g. GABA, glutamate, acetylcholine) depending on the neuronal subtype. Neurons do not express the markers nestin and double cortin.

The term "dopaminergic cells" designates cells that are characterized by expression of the marker protein tyrosine hydroxylase (TH). Other markers characteristic of dopaminergic cells include dopamine transporter (DAT) and EN1 (engrailed homolog 1). With respect to electrophysiology, completely differentiated dopaminergic neurons typically show hyperpolarization-activated action currents (Ih) (See also Storch A et al. J Chem Neuroanat. 26(2):133-142, 2003, the disclosure of which is incorporated herein by reference).

"Astrocytes" express the marker "glial fibrillary acidic protein" (GFAP), whereas "oligodendrocytes" express galactocerebrosidase (GalC).

**Hyperpolarization** is any change in a cell's membrane potential that makes it more polarized. In other words, hyperpolarization is an increase in the absolute value of a cell's membrane potential. Thus, any change of membrane voltage in which the membrane potential moves farther from zero, in either a positive or negative direction, is a hyperpolarization. **Depolarization** is a decrease in the absolute value of a cell's membrane potential. Thus, changes in membrane voltage in which the membrane potential becomes less positive or less negative are both depolarizations.

The term "uracil nucleotide" as used herein includes uridine 5'-triphosphate (UTP), uridine 5'-diphosphate (UDP) and uridine monophosphate (UMP). UTP and UDP are the preferred uracil nucleotides in the present invention.

According to the present invention "proliferation" is to be understood as a process of reproduction and/or division of cells. Therefore proliferation is the expansion of a population of cells by the continuous division of single cells into two identical daughter cells.

In the sense of the present invention "differentiation" is to be understood as a process whereby an unspecialized cell acquires the features of a specialized cell such as a neural cell. It is possible to manipulate differentiation ("directed differentiation") in that the stem cell culture conditions are optimized. Therefore differentiation is the development of cells with specialized structure and function from unspecialized precursor cells and - in general - differentiation of cells leads to a decrease in proliferation. Differentiation according to the present invention is preferably differentiation into dopaminergic cells and/or into neuronal cells.

### Depolarization and Hyperpolarization

During the hyperpolarization step the absolute value of the membrane potential of the cells may be greater than 70 mV, preferably greater than 75 mV, more preferably greater than 80 mV, most preferably greater than 85 mV. The actual membrane potential will in most cases be negative, i.e. it will usually be less than -70 mV, preferably less than -75 mV, more preferably less than -80 mV, most preferably less than -85 mV.

Preferably, the hyperpolarization step causes an increase of the absolute value of the membrane potential of the neuronal cell or NPC by at least 5 %, preferably by at least 10 %, more preferably by at least 15 %, most preferably by at least 20 % or even at least 25% or at least 50%. The hyperpolarization step may cause a membrane potential of less than -70 mV, preferably of less than -75 mV, more preferably of less than -80 mV, most preferably of less than -85 mV.

In one aspect of the invention, the hyperpolarization step therefore comprises culturing the cells obtained in step (a) in a hyperpolarization medium containing Na⁺ ions and/or at least one inhibitory neurotransmitter at a concentration sufficient to cause an increase in the absolute value of the membrane potential of the cells by at least 5 %, preferably by at least 10 %, more preferably by at least 15 %, most preferably by at least 20 % or even at least 25% or at least 50%.

In one embodiment of the invention, the hyperpolarization is achieved by culturing the neuronal cells or NPCs in a cell culture medium containing sodium ions (Na⁺) at a concentration of from about 120 mM to about 160 mM. Preferably, the concentration of sodium ions in the culture medium ranges from about 125 mM to about 155 mM, more preferably from about 130 mM to about 150 mM, most preferably from about 130 mM to about 145 mM.

The culture medium for hyperpolarization may further contain potassium ions (K⁺) at a concentration of from about 0 to about 8 mM, preferably from about 1 to about 7 mM, more preferably from about 2 to about 6 mM, still more preferably from about 3 to about 5 mM, most preferably about 4 mM.

The culture medium for the hyperpolarization step may further comprise magnesium ions (Mg²⁺) at a concentration of from about 0 to about 5 mM, preferably from about 0.3 to about 2 mM, still more preferably from about 0.5 to about 1 mM, most preferably from about 0.6 to about 0.8 mM, e.g. about 0.7 mM.

The culture medium for hyperpolarization may further comprise calcium ions (Ca²⁺) at a concentration of from about 0 to about 2 mM, preferably from about 0.5 to about 1.5 mM, most preferably from about 0.8 to about 1.2 mM, e.g. about 1 mM.

The culture medium for hyperpolarization further comprises chloride ions (Cl⁻) at a concentration of from about 50 to about 200 mM, preferably from about 100 to about 150 mM, more preferably from about 110 to about 140 mM, most preferably from about 110 to about 120 mM.

A preferred medium for hyperpolarization comprises 0.5 to 1 mM Mg²⁺, 3 to 5 mM K⁺, 0.5 to 2 mM Ca²⁺, 130 to 140 mM Na⁺ and 110 to 140 mM Cl⁻. The ions may be contained in a typical culture medium for culturing NPCs or neuronal cells (see infra).

The time period for which the neuronal cells are to be cultured in the hyperpolarization medium may range from 1 minute to several months, e.g. from 1 hour to 90 days, preferably from 24 hours to 60 days, more preferably from 48 hours to 30 days. Usually, the neuronal cells are cultured in the hyperpolarization medium for about 5 days to about 20 days, preferably for about 7 days to about 15 days.

Alternatively, hyperpolarization may be achieved by exposing the neuronal cells to an inhibitory neurotransmitter. Inhibitory neurotransmitters are known in the art and include, but are not limited to glycine, norepinephrine and GABA. The concentrations in which the inhibitory neurotransmitters may be added to the medium can be adjusted by the person skilled in the art so as to obtain the desired hyperpolarization effect. The time for which the neuronal cells may be exposed to the inhibitory neurotransmitter are the same as those described herein above in connection with the hyperpolarization medium containing an increased concentration of sodium ions.

In one embodiment, the hyperpolarization step is preceded by a depolarization step. This further enhances the maturation of the neurons which can be seen from elevated sodium currents and an improved capability of the neurons to fire action potentials. According to this embodiment, the invention relates to a method for generating mature neuronal cells, comprising (1) expanding and/or differentiating NPCs, (2) subjecting the cells obtained from step (1) to a depolarization step, and (3) subjecting the cells obtained from step (2) to a hyperpolarization step.

During the depolarization step the absolute value of the membrane potential of the cells is less than 65 mV, preferably less than 60 mV, more preferably less than 50 mV, still more preferably less than 40 mV, even more preferably less than 25 mV, most preferably less than 10 mV. Usually, the membrane potential of the cells will be higher than -70 mV, preferably higher than -60 mV, more preferably higher than -50 mV, still more preferably higher than -40 mV, even more preferably higher than -25 mV, most preferably higher than -10 mV. The depolarization step usually causes the absolute value of the membrane potential of the neuronal cells to decrease by at least 10 %, preferably by at least 20 %, more preferably by at least 50 %, most preferably by at least 75 %.

The depolarization step comprises in one embodiment the culturing of the neuronal cells in a culture medium containing K⁺ ions at a concentration of at least 8 mM, preferably at least 10 mM, more preferably at least 15 mM, most preferably at least 20 mM. Usually, the concentration of potassium ions in the depolarization medium ranges from about 8 mM to about 50 mM, preferably from about 10 mM to about 40 mM, more preferably from about 15 mM to about 30 mM, most preferably from about 20 mM to about 25 mM, e.g. about 22 mM.

The depolarization medium may further comprise Mg²⁺ ions at a concentration of from about 5 mM to about 20 mM, preferably from about 8 mM to about 15 mM, more preferably from about 10 mM to about 15 mM, e.g. about 12 mM.

The depolarization medium may further comprise Ca²⁺ ions at a concentration of from about 2 mM to about 10 mM, preferably from about 3 mM to about 8 mM, more preferably from about 4 mM to about 6 mM, e.g. about 5 mM.

The depolarization medium usually further comprises Na⁺ ions at a concentration of from about 10 mM to about 110 mM, preferably from about 25 mM to about 100 mM, more preferably from about 50 mM to about 90 mM, most preferably from about 60 mM to about 80 mM, e.g. about 70 mM.

The depolarization medium may further comprise Cl⁻ ions at a concentration of from about 20 mM to about 100 mM, preferably from about 30 mM to about 90 mM, more preferably from about 40 mM to about 80 mM, most preferably from about 60 mM to about 80 mM, e.g. about 75 mM.

A preferred depolarization medium in accordance with this invention comprises 10 to 15 mM Mg²⁺, 15 to 25 mM K⁺, 4 to 6 mM Ca²⁺, 50 to 80 mM Na⁺ and 70 to 80 mM Cl⁻.

The period of time for which the cells obtained in step (a) are cultured in the depolarization medium is the same as indicated above for the hyperpolarization step. However, if depolarization and hyperpolarization are carried out successively, the periods of time for both steps may be the same or different, they may vary independently of each other.

In another embodiment, the depolarization step may comprise exposing the cells obtained in step (a) to an excitatory neurotransmitter. Excitatory neurotransmitters are known to those of skill in the art and include but are not limited to glutamate, acetylcoline and GABA. The concentrations of the excitatory neurotransmitters can be adjusted by those of ordinary skill so as to obtain the desired depolarization effect.

The hyperpolarization step and the optional prior depolarization step is/are preferably carried out after at least partially differentiating neural progenitor cells into neuronal cells (e.g. into dopaminergic cells, GABAergic cells, serotoninergic cells, cholinergic cells). It is also possible, however, to conduct the hyperpolarization step and the optional prior depolarization step simultaneously with the differentiation of the neuronal progenitor cells into neuronal cells. For example, the neuronal progenitor cells may be cultured in a suitable differentiation medium containing differentiation inducing factors. After some time of differentiation or even in the beginning of differentiation, the ion concentrations in the medium may be adjusted such that they meet the criteria as defined above for the hyperpolarization step and the optional depolarization step. This can be done by changing the media or just adding ion solutions.

Methods and protocols for differentiating NPCs into neuronal cells are described in, e.g. WO 00/78931 A2, the contents of which is incorporated herein by reference in its entirety. Preferred methods of expanding NPCs and differentiating them into dopaminergic neuronal cells are described infra.

### Method for generating dopaminergic neuronal cells

In order to generate dopaminergic neurons from NPCs it is crucial, that the physiological pathways that control dopaminergic neurogenesis *in vivo* also can be maintained *in vitro.* The development of midbrain dopaminergic neurons, which starts in the mouse approximately at embryonic day 8, is controlled by a variety of secreted and transcription factors (Prakash and Wurst, J Physiol 2006). The onset of dopaminergic neurogenesis in the human embryo is not known but is expected to start within the second month. Dopaminergic development is controlled along important signalling centers along the anterior-posterior (AP and the dorso-ventral (DV) axes. Along the DV axis, the lipid-modified glycoprotein sonic hedgehog (SHH) is secreted by floor plate cells. Shh acts on specific receptors (Gli1) and controls the expression of various regulators of transcription. Along the AP axis, fibroblast growth factor 8 and wnt-1 are important growth factors and mitogens that also control a variety of transcription factors that belong to the homeodomain or helix-loop-helix families (Prakash and Wurst, J Physiol 2006). In addition to Shh and Fgf8, which are widely expressed other (asymmetric) genes are important to specify the dopaminergic phenotype. Among these, the growth factor wingless-1 (wnt-1) and the transcription factors Lmx1a/b, Msx1 and Otx2 seem to be most important. Other marker genes of the midbrain-hindbrain boundary which are also controlled by Fgf8 and wnt-1 are Pax2/5 and engrailed ½. The function of the latter genes for dopaminergic development are less well understood. An important downstream target of wnt-1 and Lmx1 is Otx2 which in turn inhibits the repressor of transcription Nkx2-2. Activation of Nkx 2-2 complete blocks dopaminergic neurogenesis. On the other hand, Msx1 inhibits another repressor, Nkx 6-1, promoting neuronal development. This effect is most likely mediated via activation of Neurogenin 2 (Ngn2).

Further downstream of the above mentioned genes are Engrailed 1,2 (Eng 1,2), Pitx3 and Nurr1 which then stimulate the expression of genes specific for dopaminergic neurons such as aldehyde dehydrogenase 1A1 (Aldh 1A1), tyrosine hydroxylase (TH), vesicular monoamine transporter 2 (Vmat2), dopamine D2 receptor (DRD2) and the dopamine transporter (DAT).

It was found that it is crucial that NPCs which are determined to differentiate into dopaminergic neurons are derived from tissue that expresses early as well as late markers of dopaminergic development (wnt-1 through DAT). However, NPCs during proliferation should not express late markers of dopaminergic development (Aldh 1A1, TH, DAT) but maintain high level expression of early (mitotic) markers such as wnt-1, Lmx1 a, Msx1, Ngn2 and Otx2. In addition, expression of Eng 1 should be present. Following differentiation, there should be a shift towards the markers of postmitotic dopaminergic neurons.

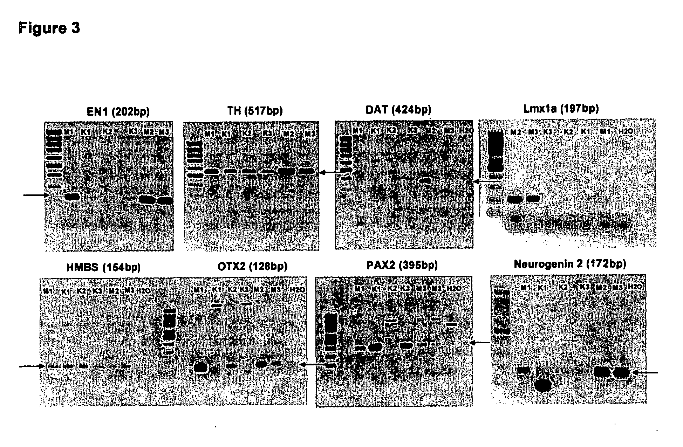

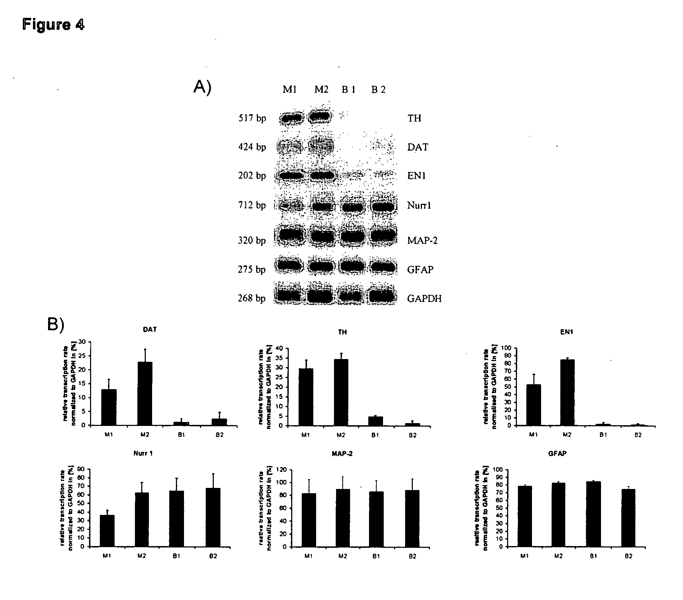

The present invention ensures that only tissue is entered into the process that includes cells at all stages of dopaminergic development. Cells expressing early markers are the "target population". However, more advanced stages are warranted to ensure dopaminergic commitment. Figures 3 and 4 indicate that midbrain samples of primary tissue from which midbrain specific NPCs are to be isolated express a variety of markers.

It has been found that human midbrain derived primary tissue which expresses all markers of dopaminergic development can efficiently be converted into a population of dopaminergic neurons by a specific differentiation protocol. The new protocol allows the generation of a unique population of dopaminergic neurons. This population of dopaminergic neuronal cells differs from known populations of dopaminergic neuronal cells derived from precursor cells in such, that they express many genes that physiologically occur in midbrain dopaminergic precursor cells during brain development. The current invention, therefore, allows for generating dopaminergic neurons that model (in vitro) physiological brain development in the living individual.

This invention relates to a method for generating dopaminergic neurons, comprising exposing neural precursor cells to forskoline, interleukin 1, FGF-8, at least one uracil nucleotide, dexamethasone and a glycogen synthase kinase 3 (GSK-3) inhibitor such as kenpaullone or 6-bromoindirubin-3'-oxime (BIO). The method of the invention comprises (i) expanding a first population of cells comprising neural precursor cells by exposing the cells to at least one growth factor so as to obtain a second population of cells, and (ii) exposing the second population of cells to forskoline, interleukin 1, FGF-8, at least one uracil nucleotide, dexamethasone and at least one GSK-3 inhibitor (e.g. kenpaullone and/or BIO) to obtain a third population of cells comprising dopaminergic neuronal cells. The details of this method of the invention are described *infra.* This method for generating dopaminergic neurons may or may not be combined with the above-described hyperpolarization step and the optional depolarization step.

This third population of cells may thereafter be exposed to a medium that leads to depolarization and excitation of neuronal precursor cells with a human neural stem cell origin. This excitation step is modelled via supplementation of potassium (> 10 mM) or excitatory neurotransmitters (glutamate, nicotine). These partially differentiated cells will finally be exposed to media that induce hyperpolarization of neuronal precursor cells. This final step of maturation is required to render cells fully mature, i. e. fire action potentials, express a variety of markers of mature neurons. The two latter steps can be performed in the presence of small molecules (nucleotides, foskolin, etc.) growth factors or cytokines.

### NPCs and their preparation

Isolation and culturing of NPCs from rodent brain has been reported in the state of the art (Daadi und Weiss, Generation of tyrosine hydroxylase producing neurons from precurors of the embryonic and adult forebrain, J Neurosci, 19:4484-4497, 1999; Liepelt et al., Differentiation potential of a monoclonal antibody-defined neural progenitor cell population isolated from prenatal rat brain by fluorescence-activated cell sorting, Brain Res Dev Brain Res, 51:267-278, 1999).

NPCs can be isolated from fetal (embryonic) or adult brain or spinal cord preparations. If an adult donor is used NPCs are preferably isolated from subventricular or hippocampal brain regions. In case of humans, the term "adult" preferably refers to an individual at an age of at least about 16 years, e.g., about 16 years to about 45 years, more preferably at least about 18 years, e.g., about 18 years to about 30 years. However, also individuals at an age of less than 16 years or greater than 45 years may be suitable donors.

NPCs were successfully isolated from various parts of brain. NPCs are abundant in fetal (embryonic) brain tissue. Preferably, NPCs are isolated from fetal midbrain tissue. Most efficiently, NPCs are prepared from human fetal brain tissue, 3 - 25 weeks of gestation, preferably 5 - 11 weeks of gestation.

Isolation of NPCs from human fetal brain tissue has been described e.g. in Buc-Caron, Neuroepithelial progenitor cells explanted from human fetal brain proliferate and differentiate in vitro, Neurobiol Dis, 2:37-47, 1995; Svendsen CN et al., Long-Term Survival of Human Central Nervous System Progenitor Cells Transplanted into a Rat Model of Parkinson's Disease, Exp Neurol 148:135-146, 1997; Sah et al, Biopotent progenitor cell lines from the human CNS ,Nat Biotechnol 15:574-580, 1997; Chalmers-Redman et al, In vitro propagation and inducible differentiation of multipotential progenitor cells from human fetal brain, Neuroscience 76:1121-1128, 1997; Schwarz SC et al. Parkinsonism Relat Disord. 12(5):302-308, 2006. The disclosure of these documents is incorporated herein by reference. The technique of preparation of brain tissue and isolation of NPCs can be adapted from these protocols.

It is also possible that the NPCs are generated from other stem cells, e.g. mesenchymal stem cells or embryonic stem cells. The mesenchymal cells can be isolated from bone marrow, preferably adult bone marrow, more preferably human adult bone marrow (e.g. bone marrow stromal cells). Alternatively, the mesenchymal stem cells can be isolated from umbilical cord blood, preferably human umbilical cord blood, or from adipose tissue. Methods of isolating mesenchymal stem cells from various sources are known to those skilled in the art. They can be converted to NPCs by methods known in the art (see e.g. WO 2005/017132 A1 filed by NeuroProgen GmbH Leipzig, and references cited therein; Hermann et al. J Cell Sci. 117(Pt19):4411-4422, 2004). Using these sources of stem cells, it is possible to use autologous cells in transplantation by isolating stem cells from the body of an individual (e.g. a person suffering from Parkinson's Disease), converting the stem cells into NPCs, culturing the NPCs as described in this application, and transplanting the autologous cells into the same individual. This embodiment has the great advantage that immunological complications such as rejection of the grafted cells can be avoided.

The method according to the present invention may further comprise the steps of isolating the NPCs from a suitable source (see supra), dissociating the NPCs into single cell suspension and/or propagating the NPCs in a suitable culture medium. These steps may be carried out prior to exposing the NPCs to the differentiation medium. Suitable culture media for culturing NPCs include but are not limited to DMEM/F12 and are described infra in the Examples.

The NPCs may be cultured under an atmosphere having an oxygen concentration of less than 20% (v/v). Preferably, the oxygen concentration is less than 15% (v/v), more preferably less than 10% (v/v), still more preferably less than 5% (v/v), most preferably about 3% (v/v). The minimum oxygen concentration may be 0.1% (v/v) or 1% (v/v). The cells may be cultured under an atmosphere of 5% (v/v) CO₂, 92% (v/v) N₂ and 3±2 % (v/v) O₂. The culturing under an reduced oxygen may be performed at any time of the culturing, for example when proliferating and/or when differentiating the NPCs as described herein.

According to a specific embodiment of the method of the invention the culture medium contains an effective Ca²⁺ concentration of less than 1.0 mmol/l, preferably less than 0.5 mmol/l, more preferably less than 0.1 mmol/l. Preferably, the total concentration of Ca²⁺ ions in the culture medium is identical to the effective concentration. If needed, a masking of Ca ions through suitable masking agents which decrease the concentration of free Ca ions may occur. For this purpose, chelating agents like EGTA, EDTA, Kronenether and others may be used. If desired, the culture medium may be free of Ca²⁺ ions apart from unavoidable contaminations; preferably, the medium is not Ca²⁺ free. A minimum amount of Ca²⁺ ions of 0.001 to 0.1 mmol/l, particularly 0.01 or 0.05 to 0.1 mmol/l culture medium, may be employed. Furthermore, the culture medium may comprise Mg ions in only low concentration, or it is free of Mg ions, apart from unavoidable contaminations. The Mg concentration of the culture medium can be less than 2 mmol/I culture medium, preferably less than 0.6 mmol/I or less than 0.1 mmol/I culture medium (See WO03/010304 A2 by NeuroProgen Leipzig GmbH). The indicated calcium ion concentrations may be present at any time of the culturing, for example when proliferating and/or when differentiating the NPCs as described herein.

It is further preferred that the NPCs are cultured at a temperature of about 35-39°C, more preferably at about 36-38°C, most preferably at about 37°C.

According to this invention, the first population of cells comprising NPCs express markers of dopaminergic development, preferably they express all markers of dopaminergic development.

The expression of the midbrain marker EN1, relative to the expression of the housekeeping gene GAPDH, is preferably at least 0.5, preferably at least 0.6, more preferably at least 0.7, still more preferably at least 0.8, as measured by quantitative real time RT PCR. A relative expression of 1 which corresponds to the value 100%, means that the level of expression as determined by quantitative real time RT PCR is equal to that of the control gene, e.g. GAPDH. The determination of expression levels may be carried out as described in the examples infra.

The expression of the marker PCNA, relative to the expression of the housekeeping gene HMBS, may range from 1 to 5, preferably from 3 to 5. The expression of the marker Wnt-1, relative to the expression of the housekeeping gene HMBS, may range from 0.5 to 10, preferably from 3 to 7. The expression of the marker Lmx1a, relative to the expression of the housekeeping gene HMBS, may range from 0.1 to 10, preferably from 0.5 to 5. The expression of the marker Lmx1b, relative to the expression of the housekeeping gene HMBS, may range from 0 to 1. The expression of the marker Msx1, relative to the expression of the housekeeping gene HMBS, may range from 1 to 1,000, preferably from 10 to 100. The expression of the marker Otx2, relative to the expression of the housekeeping gene HMBS, may range from 10 to 500, preferably from 20 to 250. The expression of the marker Ngn2, relative to the expression of the housekeeping gene HMBS, may range from 0.1 to 1,000, preferably from 1 to 10. The expression of the marker Pax 2, relative to the expression of the housekeeping gene HMBS, may range from 0 to 100, preferably from 0 to 10. The expression of the marker EN 1, relative to the expression of the housekeeping gene HMBS, may range from 0.1 to 100, preferably from 1 to 10. The expression of the marker Nestin, relative to the expression of the housekeeping gene HMBS, may range from 10 to 100,000, preferably from 100 to 1,000. The expression of the marker SOX1, relative to the expression of the housekeeping gene HMBS, may range from 1 to 100, preferably from 10 to 100. The expression of the marker SOX2, relative to the expression of the housekeeping gene HMBS, may range from 1 to 100, preferably from 10 to 100. The expression of the marker SOX10, relative to the expression of the housekeeping gene HMBS, may range from 0.1 to 100, preferably from 0.5 to 50. The expression of the marker NeurD1, relative to the expression of the housekeeping gene HMBS, may range from 0.1 to 100, preferably from 1 to 10. The expression of the marker Notch1, relative to the expression of the housekeeping gene HMBS, may range from 1 to 1,000, preferably from 50 to 250. The expression of the marker GFAP, relative to the expression of the housekeeping gene HMBS, may range from 0.1 to 5, preferably from 0.7 to 2. The expression of the marker MAP-2, relative to the expression of the housekeeping gene HMBS, may range from 0.1 to 5, preferably from 0.7 to 2. The expression of the marker Nurr1, relative to the expression of the housekeeping gene HMBS, may range from 0.1 to 1, preferably from 0.5 to 0.7. The expression of the marker Aldh 1A1, relative to the expression of the housekeeping gene HMBS, may range from 0.1 to 100, preferably from 0.5 to 50. The expression of the marker TH, relative to the expression of the housekeeping gene HMBS, may range from 0.01 to 2, preferably from 0.05 to 1.5. The expression of the marker DAT, relative to the expression of the housekeeping gene HMBS, may range from 0.1 to 2, preferably from 0.2 to 1.5. The expression of the marker DRD2, relative to the expression of the housekeeping gene HMBS, may range from 0.01 to 10, preferably from 1 to 3. The expression of the marker DRD3, relative to the expression of the housekeeping gene HMBS, may range from 0.01 to 10, preferably from 1 to 3. The expression of the marker Ptx3, relative to the expression of the housekeeping gene HMBS, may range from 0.01 to 1, preferably from 0.1 to 1. The expression of the marker Vmat2, relative to the expression of the housekeeping gene HMBS, may range from 0.01 to 1, preferably from 0.1 to 1.

The above embodiments referring to HMBS may be applied to GAPDH *mutatis mutandis.*

**Table 1: Expression of markers, relative to expression of HMBS, by the first population of cells prior to proliferation, as determined by quantitative RT PCR or realtime PCR.**

| **Marker** | **range** | **preferred range/value** |
|---|---|---|
| PCNA | 1 - 5 | 3- 5 |
| Wnt-1 | 0.5-10 | 2-8 |
| Lmx1a | 0.1 - 10 | 0.5 - 5 |
| Lmx1b | 0-1 | 0-1 |
| Msx1 | 1 - 1,000 | 10 - 100 |
| Otx2 | 10 - 500 | 20 - 250 |
| Ngn2 | 0.1 - 1,000 | 1 - 10 |
| Pax2 | 0 - 100 | 0 - 10 |
| EN 1 | 0.1-100 | 1-10 |
| Nestin | 10 - 100,000 | 100 - 1,000 |
| SOX1 | 1 - 100 | 10 - 100 |
| SOX2 | 1 - 100 | 10 - 100 |
| SOX10 | 0.1 - 100 | 0,5 - 50 |
| NeurD1 | 0.1 - 100 | 1 - 10 |
| Notch1 | 1 - 1,000 | 50 - 250 |
| GFAP | 0.1 - 5 | 0.7-2 |
| MAP-2 | 0.1-5 | 0.7-2 |
| Nurr1 | 0.1-1 | 0.5-0.7 |
| Aldh 1A1 | 0.1 - 100 | 0.5-50 |
| TH | 0.01-2 | 0.05-1.5 |
| DAT | 0.1-2 | 0.2-1.5 |
| DRD2 | 0.01 - 10 | 1 - 3 |
| DRD3 | 0.01 - 10 | 1 - 3 |
| Ptx3 | 0.01 - 1 | 0.1 - 1 |
| Vmat2 | 0.01 - 1 | 0.1 - 1 |

### Proliferating the NPCs

The method comprises proliferating the NPCs present in the first population of cells by exposing them to at least one growth factor.

Generally, growth factors in the sense of the present invention are proteins or peptides that are capable of stimulating cell division and/or differentiation. Most growth factors are proteins. When the growth factor is produced in a laboratory the growth factor has preferably the biological activity of a native mammalian growth factor which means having at least one activity of a native mammalian growth factor such as binding to the same receptor as a particular native mammalian growth factors and/or eliciting proliferation and/or differentiation and/or changes in cell size. Preferably the laboratory growth factor binds to the same receptor as a particular native mammalian growth factor. The term growth factor encompasses analogs which are deletional or substitutional mutants of a native mammalian growth factor. Furthermore, the term growth factor encompasses the growth factor from other species and naturally occurring in synthetic variants thereof. Exemplary growth factors include platelet-derived growth factor (PDGF), epidermal growth factor (EGF), vascular endothelial growth factor (VEGF), erytrhopoeitin, insulin-like growth facor-1 and -2 (IGF-1, IGF-2), transforming growth factors (TGF; TGF;), acidic and basic fibroblast growth factors (a-FGF/FGF-2, b-FGF/FGF-2), Interleukins 1,2,6 and 8 (IL-1, IL-2, IL-6, IL-8), nerve growth factor (NGF), brain-derived neurotrophic factor (BDNF), neurotrophin-3 (NT-3), interleukin-3, hematopoietic colony stimulating factors (CSFs), amphiregulin, interferon- (INF-), thyrotropin releasing hormone (TRH), and prolactin. EGF is particularly known as a 6045 Da protein with 53 amino acid residues.

Most preferred for proliferation are EGF, FGF-2 and combinations thereof.

It should be noted that also variants analogs of these agents (growth factors) which share a substantial identity with a native mammalian growth factor as listed above and which are capable of binding to receptors of the native mammalian growth factor are referred to as growth factors in the sense of the present invention. For example EGF variants or analogs which share a substantial identity with a native mammalian EGF and are capable of binding to receptor of a native mammalian growth factor can be used in the present application. These EGF variants and analogs include but are not limited to the recombinant and modified EGF having a deletion of the two C-terminal amino acids and a neutral amino acid substitution at position 51 such as asparagine, glutamine, serine or alanine (particularly EGF51N or EGF51Q) having asparagine (N) or glutamine (Q) at position 51 respectively; WO 03/040310); the EGF mutein (EGF-X16) in which the His residue at position 16 is replaced with a neutral or acidic amino acid (US Patent No. 6,191,106); the 52-amino acid deletion mutant of EGF which lacks the amino terminal residue of the native EGF (EGF-D); the EGF deletion mutant in which the amino terminal residue as well as the two C-terminal residues (Arg-Leu) are deleted (EGF-B); the EGF-D in which the Met residue at position 21 is oxidized (EGF-C); the EGF-B in which the Met residue at position 21 is oxidized (EGF-A); heparin-binding EGF-like growth factor (HG-EGF); betacellulin; amphiregulin; neuregulin; or a fusion protein comprising any of the above. Other EGF analogs or variants are described in WO 03/040310 and US Patent Nos. 6,191,106 and 5,547,935.

Fibroblast growth factor (FGF) is a family of proteins that are involved in proliferation and differentiation of several cell types. It binds to receptors belonging to the tyrosine kinase family. FGF-2 is the prototype of the fibroblast growth factor family which currently has up to 18 members of structurally related proteins. It acts as a mitogen or proliferative signal for several cell types including fibroblasts smooth muscle cells and endothelial cells and is also involved in the angiogenic process and in the formation of mesenchyme.

According to the present invention for proliferation of the NPCs, EGF and FGF-2 are particularly preferred growth factors. EGF and FGF-2 are commercially available.

According to the present invention the growth factor(s) are preferably present in a concentration in the culture medium of from 0.2 ng/ml to 2 µg/ml, more preferably 2 ng/ml to 200 ng/ml, still more preferably 10 ng/ml to 100 ng/ml, most preferably about 20 ng/ml. The concentration of FGF-2 in the culture medium is preferably 0.2 ng/ml to 2 µg/ml, more preferably 2 ng/ml to 200 ng/ml, still more preferably 10 ng/ml to 100 ng/ml, most preferably about 20 ng/ml. The concentration of EGF in the culture medium is preferably 0.2 ng/ml to 2 µg/ml, more preferably 2 ng/ml to 200 ng/ml, still more preferably 10 ng/ml to 100 ng/ml, most preferably about 20 ng/ml.

The NPCs may be cultured in the presence of EGF and FGF-2 for at least 2 days, preferably for at least 10 days, most preferably for at least 14 days, e.g., for 20 to 70 days. In one aspect, the NPCs are cultured in the presence of EGF and FGF-2 until neurosphere formation is observed, e.g., for 10 to 20 days. These neurospheres may be further expanded in medium containing EGF and FGF-2 for 1 to 15 days, preferably for 2 to 10 days. It is also possible, however, to culture the cells in the forms of neurospheres over longer periods of time, e.g. from 1 to 50 weeks or from 2 to 10 weeks. The NPCs may first be cultured in the form of neurospheres for a few days (e.g. for 1, 2, 3, 4, 5, 6 or 7 days). This means the cells are kept in suspension culture. This suspension culture of neurospheres may be followed by a so-called two-dimensional culture, i.e. an adherent culture wherein the cells adhere to the substrate. The adherent culture may be maintained for longer periods of time.

In a certain embodiment, the proliferation comprises exposing the NPCs to at least one uracil nucleotide and/or dexamethasone.

For the purpose of proliferation, the concentration of the uracil nucleotide in the culture medium is preferably from about 0.01 to about 5 µM, more preferably from about 0.1 to about 3 µM, most preferably from about 0.5 to about 2 µM, e.g. about 1 µM. The uracil nucleotide for proliferation is preferably UTP. It is also possible, however, to use UDP for proliferation.

The concentration of dexamethasone in the culture medium during proliferation may range from about 0.001 pM to about 500 µM, preferably from about 0.01 pM to about 200 µM, more preferably from about 0.1 pM to about 150 µM. According to a specific embodiment, the preferred concentration range is from about 0.01 nM to about 10 µM, more preferably from about 0.1 nM to about 3 µM, most preferably from about 0.1 nM to about 1 µM, e.g. about 1 nM.

The uracil nucleotide and/or dexamethasone may be applied at the beginning of the culturing period or added later on after some culturing without uracil nucleotide. The uracil nucleotide and/or dexamethasone may be renewed once a week in combination with a medium change. In yet another embodiment of the present invention the uracil nucleotide and/or dexamethasone is supplied daily during the contacting period wherein supplementation of five consecutive days from the beginning of the contacting period is particularly preferred.

The proliferation step may comprise culturing the cells under an atmosphere having an oxygen concentration of less than 20% (v/v) and/or in a culture medium containing an effective Ca²⁺ concentration of less than 1 mM, as described supra. These preferred embodiments described supra in connection with the culturing of NPCs apply to the proliferation step *mutatis mutandis.*

The period of time during which the NPCs are expanded in the presence of at least one growth factor, optionally in the presence of at least one uracil nucleotide and/or dexamethasone, is not particularly limited. It may range from 1 minute or less to several years. Preferred periods of time are from 1 hour to 1 year, more preferably from 24 hours to 20 weeks, more preferably from 5 to 30 days wherein durations between 1 week and 2 weeks are most preferred.

In a particularly preferred embodiment of the present invention the proliferation of the NPCs has a duration of from 1 day to 10 weeks, more preferably from 5 days to 5 weeks, and most preferred of from 1 week to 3 weeks, e.g. 2 weeks. The growth factor(s) may be applied to the culture medium at the beginning of the proliferation period. In another embodiment the growth factor is applied together with the uracil nucleotide but it is also possible to apply the growth factor prior to or after the uracil nucleotide application. It is also possible to first proliferate the NPCs in the presence of the one or more uracil nucleotide(s) and suitable growth factor(s), and then withdraw the uracil nucleotide(s) without withdrawing the growth factor(s). The effect of the uracil nucleotide(s) may persist for many weeks, even after removal from the culture medium.

The NPCs may be expanded to increase the number of living cells in the cell culture by a factor of at least 2, more preferably by a factor of at least 10, still more preferably by a factor of at least 100, most preferably by a factor of at least 1,000, relative to the number of cells in the starting population before the expansion step (e.g. the first population of cells). The number of cells in the cell culture may be increased by a factor of 1,000 to 100,000.

During and/or after proliferation (e.g. in the second population of cells), the level of expression of the dopaminergic marker DRD2, relative to the level of expression of the housekeeping gene GAPDH, is preferably less than 0.5, preferably less than 0.4, more preferably less than 0.3, still more preferably less than 0.25, as measured by quantitative real time RT PCR. A relative expression of 1 which corresponds to the value 100%, means that the level of expression as determined by quantitative real time RT PCR is equal to that of the control gene, e.g. GAPDH. The determination of expression levels may be carried out as described in the examples infra.

During and/or after proliferation (e.g. in the second population of cells), the level of expression of the proliferation marker PCNA, relative to the level of expression of the housekeeping gene HMBS preferably ranges from 1 to 5, preferably from 3 to 5, as measured by quantitative real time RT PCR. A relative expression of 1 which corresponds to the value 100%, means that the level of expression as determined by quantitative real time RT PCR is equal to that of the control gene, e.g. HMBS. The determination of expression levels may be carried out as described in the examples infra. Details on methods such as quantitative real time RT PCR can be found e.g. in *"A-Z of Quantitative PCR",* Bustin, S.A. (ed.), IUL Biotechnology, 2004, the content of which is incorporated herein in its entirety.

During and/or after proliferation, the level of expression of the marker Wnt-1, relative to the level of expression of the housekeeping gene HMBS, preferably ranges from 0.5 to 10, more preferably from 3 to 7. The level of expression of the marker Lmx1 a, relative to the expression of HMBS, may range from 0.1 to 10, preferably from 0.5 to 5. The level of expression of the marker Lmx1 b, relative to the expression of HMBS, may range from 0 to 1, preferably from 0.1 to 0.9. The level of expression of the marker Msx1, relative to the level of expression of HMBS, may range from 1 to 1,000, preferably from 10 to 100. The level of expression of the marker Otx2, relative to the level of expression of HMBS, may range from 10 to 50, preferably from 20 to 50. The level of expression of the marker Ngn2, relative to the level of expression of HMBS, may range from 0.1 to 1000, preferably from 1 to 10. The level of expression of the marker Pax2, relative to the level of expression of HMBS, may range from 0 to 100, preferably from 0 to 10. The level of expression of the marker EN1, relative to the level of expression of HMBS, may range from 0.1 to 100, preferably from 1 to 10. The level of expression of the marker Nestin, relative to the level of expression of HMBS, may range from 10 to 100,000, preferably from 100 to 1,000. The level of expression of the marker SOX1, relative to the level of expression of HMBS, may range from 1 to 100, preferably from 10 to 100. The level of expression of the marker SOX2, relative to the level of expression of HMBS, may range from 1 to 100, preferably from 10 to 100. The level of expression of the marker SOX10, relative to the level of expression of HMBS, may range from 0.1 to 100, preferably from 0.5 to 50. The level of expression of the marker NeurD1, relative to the level of expression of HMBS, may range from 0.1 to 100, preferably from 1 to 10. The level of expression of the marker Notch 1, relative to the level of expression of HMBS, may range from 1 to 1000, preferably from 50 to 250. The level of expression of the marker GFAP, relative to the level of expression of HMBS, may range from 0.1 to 500, preferably from 10 to 250. The level of expression of the marker MAP-2, relative to the level of expression of HMBS, may range from 0.1 to 5, preferably from 0.7 to 2. The level of expression of the marker Nurr1, relative to the level of expression of HMBS, may range from 0.1 to 10, preferably from 1 to 10. The level of expression of the marker Aldh 1A1, relative to the level of expression of HMBS, may range from 0.1 to 100, preferably from 0.5 to 50. The level of expression of the marker TH, relative to the level of expression of HMBS, may range from 0.01 to 2, preferably from 0.05 to 1.5. The level of expression of the marker DAT, relative to the level of expression of HMBS, may range from 0.01 to 2, preferably from 0.2 to 1.5. The level of expression of the marker DRD2, relative to the level of expression of HMBS, may range from 0.01 to 10, preferably from 1 to 3. The level of expression of the marker DRD3, relative to the level of expression of HMBS, may range from 0.01 to 10, preferably from 1 to 3. The level of expression of the marker Ptx3, relative to the level of expression of HMBS, may range from 0.01 to 5, preferably from 0.1 to 1. The level of expression of the marker Vmat2, relative to the level of expression of HMBS, may range from 0.01 to 1, preferably from 0.1 - 1. The level of expression of the marker Tuj1, relative to the level of expression of HMBS, may range from 0.1 to 200, preferably from 0.1 to 100. The level of expression of the marker EDNRB, relative to the level of expression of HMBS, may range from 0.1 to 100, preferably from 0.1 to 90. The level of expression of the marker PDGFRa, relative to the level of expression of HMBS, may range from 10 to 500, preferably from 100 to 500.

The above embodiments referring to HMBS may be applied to GAPDH *mutatis mutandis.*

**Table 2: Expression of markers, relative to expression of HMBS during and/or after proliferation, as determined by quantitative real time RT PCR.**

| **Marker** | **range** | **preferred range/value** |
|---|---|---|
| PCNA | 1 - 5 | 3- 5 |
| Wnt-1 | 0.5 - 10 | 2-8 |
| Lmx1a | 0.1 - 10 | 0,5 - 5 |
| Lmx1b | 0-1 | 0.1 - 0.9 |
| Msx1 | 1 - 1,000 | 10 - 100 |
| Otx2 | 10 - 50 | 20 - 50 |
| Ngn2 | 0.1 - 1,000 | 1 - 10 |
| Pax2 | 0 - 100 | 0 - 10 |
| EN1 1 | 0.1-100 | 1-10 |
| Nestin | 10 - 100, 000 | 100 - 1, 000 |
| SOX1 | 1 - 100 | 10 - 100 |
| SOX2 | 1 - 100 | 10 - 100 |
| SOX10 | 0.1 - 100 | 0.5 - 50 |
| NeurD1 | 0.1 - 100 | 1 - 10 |
| Notch1 | 1 - 1,000 | 50 - 250 |
| GFAP | 0.1-500 | 10 - 250 |
| MAP-2 | 0.1-5 | 0.7-2 |
| Nurr1 | 0.1 - 10 | 1 - 10 |
| Aldh 1A1 | 0.1 - 100 | 0.5 - 50 |
| TH | 0.01 - 2 | 0.05 - 1.5 |
| DAT | 0.01 - 2 | 0.2 - 1.5 |
| DRD2 | 0.01 - 10 | 1 - 3 |
| DRD3 | 0.01 - 10 | 1 - 3 |
| Ptx3 | 0.01 - 5 | 0.1 - 1 |
| Vmat2 | 0.01 - 1 | 0.1 - 1 |
| Tuj1 | 0.1 - 200 | 0.1 - 100 |
| EDNRB | 0.1 - 100 | 0.1 - 90 |
| PDGFRa | 10 - 500 | 100 - 500 |

Generally, the expression of a marker protein can be determined by methods known in the art, for example by quantitative real time RT-PCR or by immunocytochemistry using suitable antibodies.

### Differentation of the NPCs into dopaminergic cells

The method of the invention comprises differentiation of the NPCs into mature neurons with specific phenotypes. Particularly preferred is differentiation into dopaminergic cells.

Differentiation is usually induced by replacement of expansion media with mitogen-free media containing serum or serum analogue, optionally supplemented by forskoline, e.g. at a concentration of about 5 µM, or by neuropathiazol, e.g. at a concentration of about 10 µM. It is particularly preferred that the culture medium for differentiation contains at least one substance chosen from the group of IL-1 (e.g. IL-1β), IL-11, LIF, GDNF and FGF8 (for details and suitable concentrations see WO 00/78931 A2, the disclosure of which is incorporated herein by reference).

In one aspect of the invention, at least one uracil nucleotide is added to the culture medium to differentiate the NPCs into dopaminergic neural cells. For differentiating the NPCs a broad range of concentrations of the uracil nucleotide in the culture medium can be used. For example, the concentration of the uracil nucleotide in the culture medium may range from about 0.001 µM to about 500 µM, preferably from about 0.01 µM to about 200 µM, more preferably from about 0.5 to about 150 µM. Generally, UDP is effective already at lower concentrations of from while higher concentrations may be required when UTP is used. Accordingly, in a specific embodiment, UTP is employed as uracil nucleotide, and the preferred concentration range is from about 0.5 µM to about 150 µM, more preferably from about 1 µM to about 120 µM, e.g. about 100 µM. In another embodiment, UDP is employed as uracil nucleotide, and the preferred concentration range is from about 0.1 µM to about 50 µM, more preferably from about 0.5 µM to about 20 µM, e.g. about 1 µM to about 10 µM.

In a specific embodiment, dexamethasone is present in the culture medium for differentiating the NPCs in addition to the uracil nucleotide(s). The concentration of dexamethasone in the culture medium may be as indicated above. For differentiating the NPCs a broad range of concentrations of dexamethasone in the culture medium can be used. For example, the concentration of dexamethasone in the culture medium may be as indicated supra in connection with the expansion step.

In another specific embodiment, neuropathiazol is present in the culture medium for differentiating the NPCs. The concentration of neuropathiazol in the culture medium may range from about 0.1 µM to about 100 µM, preferably from about 1 µM to about 25 µM, most preferably the concentration is from about 5 µM to about 15 µM, e.g. about 10 µM.

Preferably, forskolin is present in the culture medium for differentiating the NPCs. The concentration of forskolin in the culture medium may range from about 0.1 µM to about 100 µM, preferably from about 1 µM to about 15 µM, most preferably the concentration is from about 2 µM to about 10 µM, e.g. about 5 µM.

Preferably, interleukin 1 (e.g. human interleukin 1) is present in the culture medium for differentiating the NPCs. The concentration of interleukin 1 in the culture medium may range from about 0.1 pg/ml to about 100 pg/ml, preferably from about 1 pg/ml to about 25 pg/ml, most preferably the concentration is from about 5 pg/ml to about 15 pg/ml, e.g. about 10 pg/ml.

Preferably, kenpaullone (9-Bromo-7,12-dihydroindo)o-[3,2-d][1]benzazepin-6(5*H*)-one) is present in the culture medium for differentiating the NPCs. The concentration of kenpaullone in the culture medium may range from about 1 nM to about 1 µM, preferably from about 25 nM to about 400 nM, most preferably the concentration is from about 100 nM to about 200 nM, e.g. about 150 nM. Alternatively, another GSK-3 inhibitor may be present in the culture medium.

Preferably, FGF-8 (e.g. human FGF-8) is present in the culture medium for differentiating the NPCs. The concentration of FGF-8 in the culture medium may range from about 0.1 ng/ml to about 100 ng/ml, preferably from about 1 ng/ml to about 25 ng/ml, most preferably the concentration is from about 5 ng/ml to about 15 ng/ml, e.g. about 10 ng/ml.

Preferably, B27 is present as additive in the culture medium for differentiating the NPCs. The concentration of B27 in the culture medium may range from about 0.5% to about 20%, preferably from about 2% to about 15%, most preferably the concentration is from about 4% to about 10%, e.g. about 6%.

In a special embodiment, a specific P2Y1 receptor antagonist is present in the culture medium for differentiating the NPCs. Preferably, the P2Y1 receptor antagonist is MRS 2179 (2'-Deoxy-N⁶-methyl adenosine 3',5'-diphosphate diammonium salt) which is available from e.g. Sigma-Aldrich.

The differentiation step may comprise culturing the cells under an atmosphere having an oxygen concentration of less than 20% (v/v) and/or in a culture medium containing an effective Ca²⁺ concentration of less than 1 mM, as described supra. These preferred embodiments described supra in connection with the culturing of NPCs apply to the differentiation step *mutatis mutandis.*

It is also preferred according to the present invention that the culturing method comprises proliferation as well as differentiation. In that case, proliferation and differentiation are carried out as described herein. Proliferation and differentiation can be carried out simultaneously or successively. Preferably, proliferation is carried out prior to differentiation of NPCs. It is also possible, however, to first carry out a differentiation according to the present invention, followed by a proliferation as described herein. In the latter embodiment, the differentiation is usually a partial differentiation. That means the cells are not allowed to fully differentiate into a state where they can no longer proliferate. Rather, the differentiation factor(s) are removed after a period of exposure, at a point of time when the cells are still capable of dividing but already express markers typical for dopaminergic neurons.

Partial differentiation is described in detail in WO 00/78931 A2. In accordance with this invention, the duration of exposure to the differentiation factor(s) may range from about 1 hour to several days, preferably from about 12 hours to 6 weeks, e.g. from about 6 hours to about 4 weeks.

### The mature neuronal cells of the invention

One aspect of this invention is a mature neuronal cell obtainable by the methods described herein. Preferably, the mature neuronal cell is a dopaminergic cell. Preferably, the mature neuronal cell is capable of producing action potentials. They preferably show hyperpolarization-activated action currents (Ih) (See also Storch A et al. J Chem Neuroanat. 26(2):133-142, 2003, the disclosure of which is incorporated herein by reference).

The mature neuronal cells preferably show voltage-dependent sodium currents greater than 400 pA, preferably greater than 500 pA, more preferably greater than 600 pA, still more preferably greater than 750 pA, even more preferably greater than 900 pA, most preferably greater than 1000 pA, wherein said voltage-dependent sodium current is determined by patch-clamp analysis in the whole-cell voltage-clamp mode.

The cells can be obtained by the methods described herein. The dopaminergic cells are usually characterized by the expression of one or more of the marker proteins TH, DAT, EN1, ADH 1A1, Neuregulin-1, and Nurr1. Preferably, the dopaminergic cells express at least the marker protein TH. They do usually not express the markers nestin and double cortin. The mature neuronal cells of the invention preferably express the marker molecules MAP2, Tuj1, TH, DAT, DRD2, DRD3, Ptx3 and Nurr1.

In a particular embodiment, the cells of the invention are not derived from embryonic stem cells. In another embodiment, the mature neuronal cells and the dopaminergic neuronal cells of the invention have not been genetically modified. Preferably, the mature neuronal cells and the dopaminergic neuronal cells of the invention have not been transfected or transduced. Preferably, the population of cells according to this invention does not contain genetically modified cells or transfected cells. Genetic modification can be effected by procedures such as transfection, transduction or infection of cells with DNA, usually with recombinant DNA. Usually, the mature neuronal cells and the dopaminergic neuronal cells of the invention are not derived from immortalized cell lines but rather from primary cells.

Preferably, the dopaminergic neuronal cells of the invention are isolated cells. Preferably, the population of cells of the invention comprises human cells, more preferably it does not comprise non-human cells. The mature neuronal cells of the invention may be postmitotic cells, i.e. they will no longer undergo cell division and, accordingly, no longer proliferate.

### The population of cells comprising dopaminergic neuronal cells

Another aspect of the invention is a population of cells comprising mature neuronal cells, preferably dopaminergic neuronal cells. Preferably, at least 10%, more preferably at least 15%, most preferably at elast 20% of the cells in the population of cells are mature neuronal cells as described herein.

In a specific embodiment of the population of cells, the ratio of the level of expression of GFAP to the level of expression of MAP-2 is less than 1.25, preferably less than 1.

### 1. Ratio dopaminergic cells / total cells

It is particularly preferred that in the (third) population of cells obtainable by the method described herein at least 2 %, preferably at least 5 %, more preferably at least 10 % still more preferably at least 20%, even more preferably at least 30%, and most preferred more than 50% of the cells are dopaminergic cells, preferably dopaminergic neuronal cells.

In the population of cells obtainable by the method of this invention, at least 10 % preferably at least 20% more preferably at least 30%, and most preferred more than 50% of the cells may be positive for expression of the marker protein MAP-2.

In another embodiment, at least 20%, preferably at least 40%, more preferably at least 50%, and most preferred more than 70% of the cells in the population of cells obtainable by the method of this invention are positive for expression of β-tubulin III (TUJ1).

In the population of cells obtainable by the method of this invention, at least 2 %, preferably at least 4%, more preferably at least 8% of the cells may be positive for expression of the marker protein TH (tyrosine hydroxylase).
In another embodiment, at least 1%, preferably at least 2%, more preferably at least 4% of the cells in the population of cells obtainable by the method of this invention are positive for expression of the marker protein dopamine transporter (DAT).
In another embodiment, at least 2%, preferably at least 4%, more preferably at least 8% of the cells in the population of cells obtainable by the method of this invention are positive for expression of the marker protein EN1 (engrailed homolog 1).
In another embodiment, at least 1%, preferably at least 4%, more preferably at least 8% of the cells in the population of cells obtainable by the method of this invention are positive for expression of all of the following marker proteins: MAP-2 and TH. In a further embodiment, the cells are further positive for one or more of the marker proteins DAT and EN1.

### 2. Ratio dopaminergic cells / neuronal cells

The method of the invention allows to achieve an increase in the ratio of the number dopaminergic cells to the number of neuronal cells in the population of cells. That is, the ratio of the number of cells that are positive for a marker for dopamincergic cells to the number of cells that are positive for a marker for neuronal cells is increased by the method of the invention. For example, the ratio of the number of TH-positive cells to the number of MAP-2-positive cells can be increased.

The ratio of the number of TH-positive cells to the number of MAP-2-positive cells in the population of cells is usually at least 0.05, more preferably at least 0.15, still more preferably at least 0.25, most preferably at least 0.40
It is a particular advantage of the present invention that a high number of dopaminergic cells can be generated by the method described herein. This is a remarkable progress since it is important for transplantation purposes that a high percentage of the cells that are transplanted into a patient suffering from PD are already dopaminergic cells.

### 3. Ratio of marker expression relative to expression of housekeeping gene

After differentiation (e.g. in the third population of cells), the level of expression of the dopaminergic marker DRD2, relative to the level of expression of the housekeeping gene GAPDH, HMBS, or Histone 2X is preferably at least 1, preferably at least 1.2, more preferably at least 1.4, still more preferably at least 1.6, as measured by quantitative real time RT PCR.

After differentiation (e.g. in the third population of cells), the level of expression of the dopaminergic marker DRD3, relative to the level of expression of the housekeeping gene HMBS, is preferably at least 1, preferably at least 3.5, more preferably at least 10, still more preferably at least 15, as measured by quantitative real time RT PCR.

After differentiation, the level of expression of the marker PCNA, relative to the level of expression of HMBS, may range from 0.1 to 5, preferably from 0.1 to 1. After differentiation, the level of expression of the marker Wnt-1, relative to the level of expression of HMBS, may range from 0.1 to 5, preferably from 0.1 to 1. After differentiation, the level of expression of the marker Lmx1a, relative to the expression of HMBS, may range from 0.1 to 10, preferably from 0.1 to 9. After differentiation, the level of expression of the marker Msx1, relative to the expression of HMBS, may range from 10 to 250, preferably from 10 to 100. After differentiation, the level of expression of the marker Otx2, relative to the expression of HMBS, may range from 50 to 250, preferably from 100 to 250. After differentiation, the level of expression of the marker Ngn2, relative to the expression of HMBS, may range from 0.1 to 100, preferably from 1 to 10. After differentiation, the level of expression of the marker EN1, relative to the expression of HMBS, may range from 0.1 to 10. After differentiation, the level of expression of the marker Nestin, relative to the expression of HMBS, may range from 50 to 250, preferably from 50 to 150. After differentiation, the level of expression of the marker SOX1, relative to the expression of HMBS, may range from 5 to 100, preferably from 5 to 20. After differentiation, the level of expression of the marker SOX2, relative to the expression of HMBS, may range from 10 to 2,500, preferably from 500 to 1,000. After differentiation, the level of expression of the marker SOX10, relative to the expression of HMBS, may range from 0.05 to 20. After differentiation, the level of expression of the marker NeurD1, relative to the expression of HMBS, may range from 1 to 100, preferably from 1 to 100. After differentiation, the level of expression of the marker Notch1, relative to the expression of HMBS, may range from 10 to 250, preferably from 10 to 100. After differentiation, the level of expression of the marker GFAP, relative to the expression of HMBS, may range from 250 to 5,000, preferably from 1,000 to 5,000. After differentiation, the level of expression of the marker MAP-2, relative to the expression of HMBS, may range from 250 to 2,500. After differentiation, the level of expression of the marker Nurr1, relative to the expression of HMBS, may range from 1 to 100, preferably from 10 to 100. After differentiation, the level of expression of the marker TH, relative to the expression of HMBS, may range from 1 to 100, preferably from 10 to 100. After differentiation, the level of expression of the marker DAT, relative to the expression of HMBS, may range from 1 to 100, preferably from 10 to 100. After differentiation, the level of expression of the marker DRD2, relative to the expression of HMBS, may range from 1 to 10, preferably from 1.5 to 10. After differentiation, the level of expression of the marker DRD3, relative to the expression of HMBS, may range from 1 to 50, preferably from 10 to 50. After differentiation, the level of expression of the marker Ptx3, relative to the expression of HMBS, may range from 5 to 250, preferably from 100 to 250. After differentiation, the level of expression of the marker Tuj1, relative to the expression of HMBS, may range from 500 to 5,000. After differentiation, the level of expression of the marker EDNRB, relative to the expression of HMBS, may range from 0.5 to 10, preferably from 0.5 to 10. After differentiation, the level of expression of the marker PDGFRa, relative to the expression of HMBS, may range from 1 to 100, preferably from 1 to 99.

The above embodiments referring to HMBS may be applied to GAPDH *mutatis mutandis.*

**Table 3. Expression of markers after differentiation.**

| **Marker** | **ratio: expression of marker / expression of HMBS** | **preferred ratio: expression of marker / expression of HMBS** |
|---|---|---|
| PCNA | 0.1 - 5 | 0.1-1 |
| Wnt-1 | 0.1 - 5 | 0.1 - 1 |
| Lmx1a | 0.1 - 10 | 0.1 - 9 |
| Msx1 | 10 - 250 | 10 - 100 |
| Otx2 | 50 - 250 | 100 - 250 |
| Ngn2 | 0.1 - 100 | 1 - 10 |
| EN 1 | 0.1-10 | 0.1-9 |
| Nestin | 50 - 250 | 50 - 150 |
| SOX1 | 5 - 100 | 5 - 20 |
| SOX2 | 10 - 2,500 | 500 - 1,000 |
| SOX10 | 0.05 - 20 | 0.06 - 19 |
| NeurD1 | 1 - 100 | 1 - 90 |
| Notch1 | 10 - 250 | 10 - 100 |
| GFAP | 250 - 5,000 | 1,000 - 5,000 |
| MAP-2 | 250 - 2,500 | 260 - 2,500 |
| Nurr1 | 1 - 100 | 10 - 100 |
| TH | 1 - 100 | 10 - 100 |
| DAT | 1 - 100 | 10 - 100 |
| DRD2 | 1.0 - 10.0 | 1.5-10 |
| DRD3 | 1 - 50 | 10 - 50 |
| Ptx3 | 5 - 250 | 100 - 250 |
| Tuj1 | 500 - 5,000 | 550 - 5,000 |
| EDNRB | 0.5 - 10 | 0.5 - 10 |
| PDGFRa | 1 - 100 | 1 - 100 |

In addition to gene expression, posttranslational modifications are also relevant.- The said cells exhibit a significant increase of activation (phosphorylation) of MAP kinases, i. e. ERK1/2 and p38 by at least 50 %, preferred at least 100 % within a few hours (e.g. 2, 4 or 6 hours) following stimulation with IL-1.

In a particularly preferred embodiment, the population of cells comprises dopaminergic neurons, wherein
the level of expression by the cells of the marker MAP2 is at least 250
the level of expression by the cells of the marker Tuj1 is at least 500
the level of expression by the cells of the marker TH is at least 8,
the level of expression by the cells of the marker DAT is at least 10,
the level of expression by the cells of the marker DRD2 is at least 1.5,
the level of expression by the cells of the marker DRD3 is at least 10,
the level of expression by the cells of the marker Ptx3 is at least 100,
the level of expression by the cells of the marker SOX2 is at least 500,
the level of expression by the cells of the marker Nurr1 is at least 10, and
the level of expression by the cells of the marker Otx2 is at least 100,
wherein the level of expression by the cells of each of the marker proteins is indicated relative to the level of expression by the cells of the housekeeping gene hydroxymethylbilansynthase (HMBS). Other embodiments refer to expression of the housekeeping gene GAPDH *mutatis mutandis.*

### Use of the invention

Another aspect of the present invention is the use of the dopaminergic cells of the invention for the manufacture of a pharmaceutical composition for treating a neurodegenerative disease and/or neuronal injury. A pharmaceutical composition in the sense of the present invention is any composition which is suitable for pharmaceutical purposes and includes compositions for transplantation purposes. Compositions for transplantation purposes are particularly preferred.

A neurodegenerative disease in the sense of the present invention is a condition which affects brain function. Neurodegenerative diseases result from deterioration of neurons. They are divided into two groups: conditions causing problems with movement and conditions affecting memory and conditions related to dementia. They are usually marked by the loss of nerve cells. Examples of neurodegenerative diseases are Alzheimer disease, Creutzfeld-Jakob disease, multiple system atrophy (MSA) and Parkinson's disease.

Neuronal injury in the sense of the present invention is to be understood as for example occurring after brain-surgical operations.

According to the present invention the use of dopaminergic neuronal cells and in particular the pharmaceutical composition resulting therefrom is particularly suitable for treating neurodegenerative diseases which show a predominant loss of dopaminergic neurons such as the Parkinson's disease. Parkinson's disease (PD) is a chronic disease of the central nervous system caused by a lowered level of the inhibitory neurotransmitter dopamine. PD is a slowly progressive disease that affects a small area of cells in the midbrain known as the substantia nigra. The substantia nigra is an area in the basal ganglia of the brain. Gradual degeneration of these cells causes a reduction in the vital chemical dopamine. This decrease in dopamine is what causes the symptoms of the disease. Typical symptoms include muscular tremors and weakness as well as stiffness or rigidity of the limbs and trunk, slowness of movement, postural instability or impaired balance and coordination.

Another embodiment of the present invention is the use of at least one uracil nucleotide for the proliferation and/or differentiation of NPCs. Yet another embodiment of the invention is the use of dexamethasone for the proliferation and/or differentiation of NPCs. Proliferation and differentiation are to be understood as described hereinbefore.
**Figure 1****:** Comparison of (i) the two-step differentiation protocol comprising depolarization followed by hyperpolarization, (ii) control condition and (iii) single step differentiation procedure employing either a depolarization or a hyperpolarization step only. Depicted is the analysis of expression of several neuronal markers following differentiation by the above-described protocols. The control media comprises only removal of growth factors, forskolin and IL-1. Media are supplemeted by B27 (4 %).
**Figure 2****:** Sodium currents of individual neurons and quantification of > 6 cells differentiated either with control, depolarization or hyperpolarization media only or sequential differentiation in depolarization media, followed by hyperpolarization media.
**Figure 3****:** RT-PCR of primary cultures derived from various brain regions (M) = midbrain, (K1) = spinal cord, (K2) = retina, (K3) = unclear. Note that DAT expression is restricted to M2 but all other markers correspond to ventral midbrain patterning.
**Figure 4****:** Analysis of primary human fetal mesencephalic (M) and non-mesencephalic (B) cells by semiquantitative RT-PCR. A) Representative RT-analysis of neuronal (MAP-2), dopaminergic (TH, DAT, Nurr-1), midbrain specific (EN1) and glial genes (GFAP). Tissue samples expressing the highest amount of DAT and TH are mesencephalic (M). B) Transcription levels are expressed relative to the housekeeping gene GAPDH. Data are means ± SEM from n-3 replicates.
**Figure 5****:** Quantitative RNA analysis of dopamine D2 receptor expression in midbrain derived NPCs during expansion and after 10 days of differentiation. Note the significant increase in DRD2 expression.
**Figure 6** displays the expression of stem cells markers such as Otx2, Sox1/2 and Notch1. Only the marker of early dopaminergic neurons Otx2 is markedly increased upon differentiation. In figures 6-10, transcripts are expressed relative to the housekeeping gene HMBS. The term "proliferation" in figures 6-10 refers to a suspension of NPCs during proliferation (expansion).
**Figure 7****:** Expression of independent stem cell markers (Nestin and sx1) in differentiated cells.
**Figure 8****:** Expression analysis of stem cell receptor PDGFR and the non-specific receptor EDBNR in midbrain derived NPCs following differentiation (relative to HMBS).
**Figure 9****:** Expression analysis of GFAP and β-tub in cells differentiated according to the invention (relative to HMBS).
**Figure 10****:** Expression analysis of various markers of dopaminergic neurogenesis in cells differentiated according to the invention (relative to HMBS).

The following non-limiting examples further illustrate the invention.

### Examples

### Material and methods

### Preparation of human midbrain-derived NPCs (hmNPCs)

Human fetal midbrain tissue was used to generate hmNPCs cultures. Samples were harvested and supplied by Advanced Bioscience Resources Inc., Alameda, CA, according to NIH and local IRB guidelines. Only if there was no evidence for fetal pathology and informed consent was obtained, parts of the brain were transferred to our laboratory. The quality of the tissue was estimated by FACS analysis (dopamine transporter - DAT expression analysis, when more than 0.5% cells positive for DAT it was considered as mesencephalic) and this was confirmed by tyrosine hydroxylase (TH)- immunoreactivity. Primary cultures were analyzed by RT-PCR for markers of dopaminergic neurons (TH, DAT and EN1).

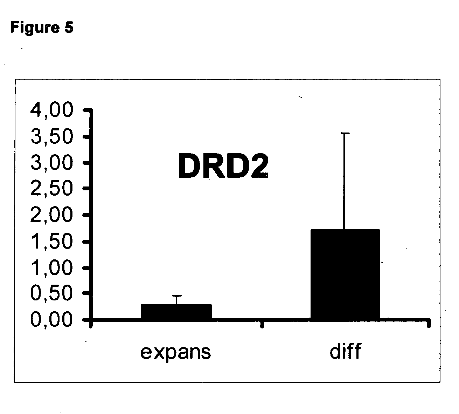

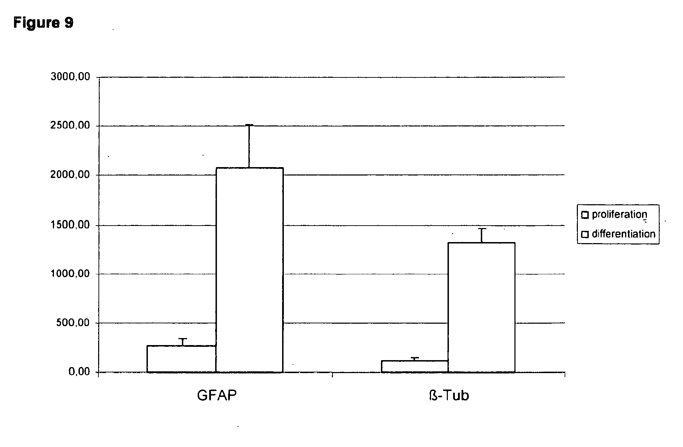

Tissue from a 10-14-week-old fetus was dissociated into single-cell suspension by incubating in 0.1 mg/ml papain / DNase solution (100µg/ml; Roche, Mannheim, Germany) for 30 min at 37°C, followed by washing with PBS, incubation in antipain (50 µg/ml; Roche) for 30 min at 37°C, and finally homogenization by gentle trituration using fire-polished Pasteur pipettes. Propagation of hmNPCs was performed in a monolayer via plating onto polyornithine-fibronectin pre-coated culture dishes at a density of 30,000 cells / cm². For **expansion** of hmNPC defined media (DMEM high glucose cat. # E15 - 843 PAA / F12 cat. # E15 - 016 PAA) without any xenogenic material (e.g. serum supplements, serum, etc.) was used, but with addition of epidermal growth factor (EGF) and fibroblast growth factor2 (FGF-2; 20 ng/ml each, both from PromoCell, Heidelberg, Germany). Cells were expanded for prolonged periods (> 10 passages) in reduced atmospheric oxygen (3 %) (Storch et al. 2001; Milosevic et al. 2005).
(A) Dopaminergic **differentiation** is achieved via application of neurobasal medium (cat. # 21103 - 049 Gibco) containing the additives B27 (6 %), Forskolin (5µM), rhu-IL1 (10 pg/ml), FGF8 (10 ng/ml), UTP (100µM), dexamethasone (1µM) and kenpaullone (150 nM). This protocol was applied to produce the data shown in Figures 5 to 10.
(B) **Hyperpolarisation** (Hyperpol.) and/or **depolarization** (Depol.) were carried out via application of neurobasal medium (cat. # 21103 - 049 Gibco) containing the additives B27 (4 %), Forskolin (5µM), rhu-IL1 (10 pg/ml), wherein the ion concentrations had been adjusted as follows:

| | **Type of medium:** | | |
|---|---|---|---|
| **Ion** | **Depol.** | **Hyperpol.** | **Control [see (A) above]** |
| [Mg²⁺] | 12.2 mM | 0.7 mM | 0.8 mM |
| [K⁺] | 21.8 mM | 4.2 mM | 5.4 mM |
| [Ca²⁺] | 5 mM | 1.05 mM | 1.8 mM |
| [Na⁺] | 68.2 mM | 135.5 mM | 78.2 mM |
| [Cl⁻] | 75.3 mM | 117 mM | 61.9 mM |

Expanded NPCs were cultured under the following different conditions:
a) in control medium for 14 days,
b) depolarization medium for 14 days,
c) in hyperpolarization medium for 14 days, or
d) in depolarization medium for 7 days, followed by hyperpolarization medium for 7 days.

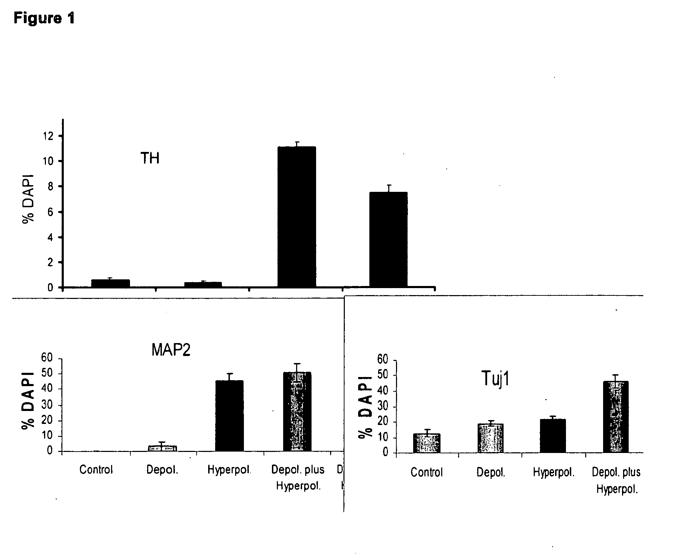

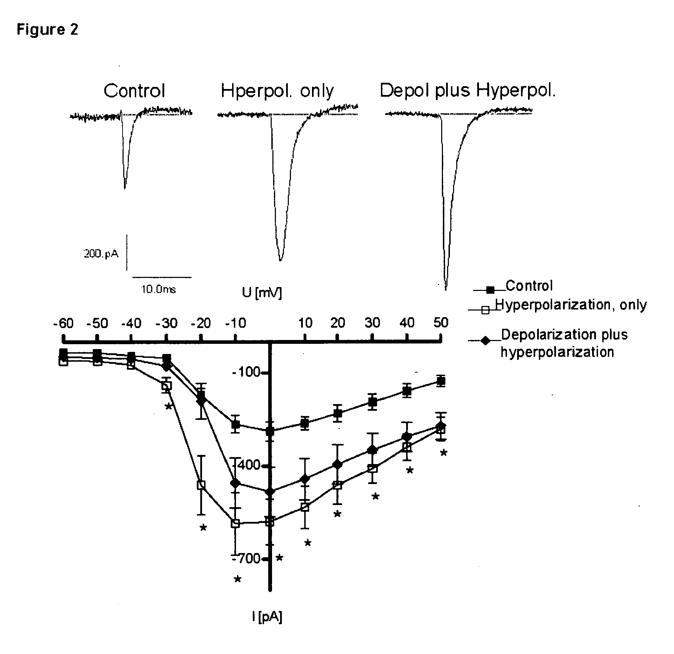

This protocol (B) was applied to produce the data shown in Figures 1 and 2.

### Electrophysiology

Patch-clamp analyses of cells were carried out (after (a) *in vitro* basic differentiation in control medium, (b) depolarization alone, (c) hyperpolarization alone, and (d) depolarization and hyperpolarization) at room temperature using an inverted microscope DMIL (Leica, Bensheim, Germany) and an EPC-9 amplifier (HEKA, Lambrecht, Germany). Recordings of voltage-gated sodium channels were obtained in the whole-cell voltage-clamp mode by stepwise depolarisations with increasing amplitudes from the holding potential of -70mV to 50mV in steps of 10mV.

Series resistance values were continuously measured during all recordings. The external bath solution contained (in mM): 142 NaCl, 1 CaCl₂, 8 KCI, 6 MgCl₂, 10 Glucose, and 10 HEPES (pH 7.4; 320 mOsm). Micropipettes were formed from thin-walled borosilicate glass (Science Products, Hofheim, Germany) with a Flaming Brown electrode puller P-97 (Sutter Instrument Co., Novato, USA) and a Micro Forge (Narishige, Tokio, Japan). Electrodes had resistances of 2-4 MΩ when filled with the internal solution containing (in mM): 153 KCI, 1 MgCl2, 10 HEPES, 5 EGTA, and 2 MgATP (pH 7.3; 305 mOsm). Whole cell currents were low-pass filtered at 2-5 kHz, digitized at 10 kHz, and analyzed using PulseFit software (HEKA, Lambrecht, Germany) and Prism 4 (GraphPad Software Inc, San Diego, USA). Numerical data were expressed as mean ± SEM. Statistical significance, using Student's t test (two tailed, unpaired), was taken as P < 0.05.

### RNA-extraction and semiquantitative RT-PCR

Total cellular RNA was extracted from hNPCs using RNeasy total RNA purification kit (Qiagen, Hilden, Germany). 50ng of total RNA was used in a RT-reaction together with oligo(dT)₂₀ primer (final concentration 5 µM) and dNTPs of a final concentration of 1 mM. After heating to 65°C for 5 minutes, the mixtures were chilled on ice and supplemented with reaction buffer and DTT (final concentration 10 mM) and 15 units reverse transcriptase (Invitrogen Life Technologies, Karlsruhe, Germany). First strand cDNA synthesis was carried out at 50°C for 60 min. Reactions were stopped by incubation at 85°C for 5 min. cDNAs were subsequently analyzed for expression of TH, DAT, EN1, MAP-2, GFAP, Nurr1, OTX2, Pax2 and Lmx1 a with GAPDH or HMBS as internal control. PCR reactions were performed with Platinum® *Taq* DNA Polymerase (Invitrogen) according to the instructions of the manufacturer. The sequences of primers are listed in the following Tables 4 and 5.

**Table 4 Primer sets**

| Gene (Protein) | Sequence (forward; reverse) | Product (bp) | SEQ ID NO: |
|---|---|---|---|
| *TH* (Tyrosine hydroxylase) | 5'-GTC CCG AGC TGT GAA GGT GTT TGA-3' | 517 | 1 |
| | 5'-ATT GTC TTC CCG GTA GCC GCT GAA-3' | | 2 |
| *DAT* (Dopamine transporter) | 5'- TCC GGC TTC GTC GTC TTC TC-3' | 424 | 3 |
| | 5'- ACA CCA TAG AAC CAG GCC ACT-3' | | 4 |
| *EN1* (Engrailed 1) | 5'- CTC GCA GCA GCC TCT CGT AT-3' | 202 | 5 |
| | 5'- CCG CTG CTC CGT GAT GTA G-3' | | 6 |
| *MAP-2* (Microtubule associated protein 2) | 5'- ATC AAG GCG GAG CAG GGG GAA GGA C-3' | 320 | 7 |
| | 5'- TGG GGG TGG AGA AGG AGG CAG ATT-3' | | 8 |
| *GFAP* (Glial fibrirally acidic protein) | 5'- GCC AAG GAG CCC ACC AAG C-3' | 275 | 9 |
| | 5'- ATC TCC TCC TCC AGC GAC TC-3' | | 10 |
| *Nurr1(Nuclear factor 1)* | 5'- CGG ACA GCA GTC CTC CAT TAA GGT-3' | 712 | 11 |
| | 5'- CTG AAA TCG GCA GTA CTG ACA GCG-3' | | 12 |
| *GAPDH (Glyceraldehyde-3-phosphate dehydrogenase)* | 5'- TCC CCA CTG CCA ACG TGT CAG TG-3' | 268 | 13 |
| | 5'- ACC CTG TTG CTG TAG CCA AAT TCG-3' | | 14 |
| *Lmx1a* (LIM homeobox transcription factor 1 alpha) | 5'- CAG GGA AAG GAA CTG CTG AG-3' | 197 | 15 |
| | 5'- CTG GTT TTG GAA CCA CAC CT -3' | | 16 |
| *Otx2* (Orthodenticle homolog 2) | 5'- CAC TTC GGG TAT GGA CTT GC -3' | 128 | 17 |
| | 5'- CGG GTC TTG GCA AAC AGT G -3' | | 18 |
| *Pax2* (Paired box gene 2) | 5'- GTA CTA CGA GAC CGG CAG CAT C -3' | 395 | 19 |
| | 5'- CGT TTC CTC TTC TCA CCA TTG G-3' | | 20 |
| *Neurogenin2* | 5'- CGC ATC AAG AAG ACC CGT AG-3' | 172 | 21 |
| | 5'- GTG AGT GCC CAG ATG TAG TTG TG-3' | | 22 |
| *HMBS* (Hydroxymethylbilane-Synthase) | 5'- TCG GGG AAA CCT CAA CAC C-3' | 154 | 23 |
| | 5'- CCT GGC CCA CAG CAT ACA T-3' | | 24 |
| MSX 1 | 5'-GCC TTC CCT TTA ACC CTC AC-3' | 235 | 25 |
| | 5'-CAG GAG ACA TGG CCT CTA GC-3' | | 26 |

Further primer sequences for detecting markers via PCR are listed in Table 5:

| **Marker (Gene, protein)** | **Sequence (forward; reverse)** | **SEQ ID NO:** |
|---|---|---|
| Lmx1a | 5'- CAG GGA AAG GAA CTG CTG AG-3' | 27 |
| | 5'- CTG GTT TTG GAA CCA CAC CT -3' | 28 |
| Msx1 | 5'-GTT TCA CCT CTT TGC TCC CTG-3' | 29 |
| | 5'-ATG GCC TCT AGC TCT GTT CAA CT-3' | 30 |
| Otx2 | 5'-CAC TTC GGG TAT GGA CTT GC-3' | 31 |
| | 5'-CGG GTC TTG GCA AAC AGT G-3' | 32 |
| Ngn2 | 5'-CGC ATC AAG AAG ACC CGT AG-3' | 33 |
| | 5'GTG AGT GCC CAG ATG TAG TTG TG-3' | 34 |
| Nestin | 5'-TGG CTC AGA GGA AGA GTC TGA-3' | 35 |
| | 5'-TCC CCC ATT TAC ATG CTG TGA-3' | 36 |
| SOX1 | 5'-GCC CAG GAG AAC CCC AAG-3' | 37 |
| | 5'-CGT CTT GGT CTT GCG GC-3' | 38 |
| Sox2 | 5'-CAG GAG AAC CCC AAG ATG C-3' | 39 |
| | 5'-GCA GCC GCT TAG CCT CG-3' | 40 |
| SOX10 | 5'-GCA AGG CAG ACC CGA AGC-3' | 41 |
| | 5'-GTC CAA CTC AGC CAC ATC AAA G-3' | 42 |
| NeuroD1 | 5'-CCG TCC GCC GAG TTT G-3' | 43 |
| | 5'-GCG GTG CCT GAG AAG ATT G-3' | 44 |
| Notch 1 | 5'-GCG ACA ACG CCT ACC TCT-3' | 45 |
| | 5'-GCA CAC TCG TAG CCA TCG-3' | 46 |
| GFAP | 5'-GAG GCG GCC AGT TAT CAG GA-3' | 47 |
| | 5'-GTT CTC CTC GCC CTC TAG CA-3' | 48 |
| MBP | 5'CGG CAA GAA CTG CTC ACT ATG-3' | 49 |
| | 5'GTG CGA GGC GTC ACA ATG-3 | 50 |
| Nurr1 | 5'-CTG TGC CCA ACC CCA TTC-3' | 51 |
| | 5'-CGC ACG CCG TAG TGT TGG-3' | 52 |
| TH | 5'-AGC TCC TGA GCT TGT CCT TG-3' | 53 |
| | 5'-TGT CCA CGC TGT ACT GGT TC-3' | 54 |
| DAT | 5'-TGC GTG CCA CAT CAA TAA CA-3' | 55 |
| | 5'-AAC ATC CTT CAC TCA GTA TTG CTA A-3' | 56 |
| DRD2 | 5'-GCA AGC GAG TCA ACA CCA-3' | 57 |
| | 5'-CGGTGCAGAGTTTCATGTCC-3' | 58 |
| DRD3 | 5'-GTC TGC TCC ATC TCC AAC C-3' | 59 |
| | 5'-TTC CGT CTC CTT TGT TTC AG-3' | 60 |
| Ptx3 | 5'-CCA CTG AGG ACC CCA CG-3' | 61 |
| | 5'-TCG TCC GTG GCT TGC-3' | 62 |
| Tuj1 | 5'-GGC CTC TTC TCA CAA GTA CG-3' | 63 |
| | 5'-CCA CTC TGA CCA AAG ATG AAA-3' | 64 |
| EDNRB | 5'TGG TTC TGG CTG TCC CTG A-3' | 65 |
| | 5'AAG CAG AAA TAG AAA CTG AAT AGC C-3' | 66 |
| PDGFRa | 5'CCT GGA GAA GTG AAA GGC AAA-3' | 67 |
| | 5'CTT TGA CCT CCC TGG TAG CC-3 | 68 |

For TH, DAT, MAP-2, GFAP, EN1, Neurogenin2, OTX2, Pax2 and HMBS PCR was carried out in 35 cycles with 45 sec denaturation at 95°C, 30 sec annealing at 60°C and 45 sec elongation at 72°C. For Lmx1 a and Nurr1 annealing temperature was elevated to 62°C and 65°C respectively and for GAPDH number of cycles was reduced to 30. PCR products were separated by agarose gel electrophoresis (1%) and visualized with 0,1% ethidium bromide under UV transillumination using DigiGenius gel documentation system (Syngene). Levels of TH, MAP-2, DAT, EN-1, GFAP and Nurr1 expression relative to GAPDH were quantified densitometrically using GeneTools software (Syngene).

### RNA extraction, RT-PCR and quantitative real-time RT-PCR analysis

Total cellular RNA was extracted from mesencephalic NPCs during expansion and differentiation using RNAeasy total RNA purification kit followed by treatment with RNase-free DNase (Qiagen, Hilden, Germany). D₃ receptor mRNAs were detected by amplification of 1 µL cDNA in a 25 µL reaction volume in Fermentas PCR buffer containing dNTP mix, 2.5 mM MgCl₂, 200 nM primers and 0.625 U Taq polymerase (5 /µL). These mRNAs were amplified parallel with glyceraldehyde-3-phosphate (GAPDH) primers. Primer sequences (forward and reverse) and lengths of the amplified products were as follows: Dopamine D₃ receptor: 5'-GCA GTG GTC ATG CCA GTT CAC TAT CAG-3' (SEQ ID NO:69) and 5'-CCT GTT GTG TTG AAA CCA AAG AGG AGA GG-3' (SEQ ID NO:70), 137 bp product (Narita et al. (2003) J Neurosci 23: 1006-1012); GAPDH: 5'-GGG TGG AGC CAA ACG GGT C-3' (SEQ ID NO:71) and 5'-GGA GTT GCT GTT GAA GTC GCA-3' (SEQ ID NO:72), 532 bp product. After initial denaturation at 94°C for 4 min, amplification of dopamine D₃ receptor was carried out for 35 cycles at the following temperatures: Denaturation at 94°C for 45 sec.; annealing at 65°C for 30 sec; extension at 72°C for 1 min. The last cycle reaction was stopped by incubation at 72°C for 10 min. GAPDH amplification was carried out for 25 cycles at the following temperatures: denaturation at 94°C for 45 sec.; annealing at 56°C for 40 sec; extension at 72°C for 1 min. The last cycle reaction was ended by incubation at 72°C for 10 min. The reaction was performed on a MJ Research PTC-200 Thermal Cycler (Bio-Rad Laboratories, Inc., Waltham, MA, USA). The PCR products were fractionated by 1.8 % agarose gel electrophoresis. The gels were then ethidium bromide-stained and photographed with a UV transilluminator equipped with CCD camera.

Quantitative real-time one step RT-PCR was carried out using the LightCycler^{®} System (Roche, Mannheim, Germany), and amplification was monitored and analyzed by measuring the binding of the fluorescence dye SYBR Green I to double-stranded DNA. 1 µl (50 ng) of total RNA was reversely transcribed and subsequently amplified using QuantiTect SYBR Green RT-PCR Master mix (Qiagen) and 0.5 µmol I⁻¹ of both sense and antisense primers. Tenfold dilutions of total RNA were used as external standards. Standards and samples were simultaneously amplified. After amplification, melting curves of the RT-PCR products were acquired to demonstrate product specificity. The results are expressed relative to the housekeeping gene *HMBS* (hydroxymethylbilane synthase). Primer sequences (forward, reverse) were as follows: dopamine D2 receptor (5'-CCT GAC AGT CCT GCC AAA CC-3' (SEQ ID NO:73), 5'-GCT TCC TGC GGC TCA TCG-3' (SEQ ID NO:74)); dopamine D3 receptor (5'- CCT ACT ACG CCC TGT CCT ACT G-3' (SEQ ID NO:75), 5'-CAC CAC CCA CGG CAT CAC-3' (SEQ ID NO:76)).

### Cell cycle analysis

Exponentially growing NPCs were prepared for the cell cycle analysis by lysing cells in 300 µl of hypotonic lysis buffer (0.1% sodium citrate, 0.1% Triton X-100, and 50 µg/ml PI). The cells were subsequently analyzed by flow cytometry, on a FACScan (Becton Dickinson, Heidelberg, Germany), using 488nm excitation, gating out doublets and clumps using pulse processing and collecting fluorescence above 620nm according to Nicoletti method. (Nicoletti et al. (1991) J Immunol Methods 139: 271-279). Histograms of DNA content were acquired using the CellQuest software. The number of nuclei present in each peak of the histogram (sub-G1, G1, S, G2/M) was analyzed by measuring the peak area. For data analysis and correction of the background noise histograms were processed with the ModFit LT software (Verity, Turramurra, Australia). Cells to the left of the G1 peak containing hypodiploid DNA were considered as apoptotic.

### Immunocytochemistry

Differentiated untreated, 7-OH-DPAT treated and raclopride + 7-OH-DPAT treated NPCs were fixed in 4% paraformaldehyde in PBS for 15 min at room temperature and washed with PBS, counterstained with the DNA-binding dye 4'-6-Diamidino-2-phenylindole (DAPI, 2 µg/ml in PBS) for 15 min at room temperature, twice washed in PBS followed by incubation in blocking buffer (10% FCS, 0,2% Triton-X 100 in PBS, pH 7.2) for 30 min at room temperature. After incubation with anti-Tuj-1 primary antibody (Covance, Berkeley,CA, USA) or anti-Doublecortin (DCX) primary antibody (Santa Cruz Biotechnology, Inc., Santa Cruz, CA) for 1 h at room temperature in blocking buffer, the cells were incubated with biotin-conjugated secondary antibodies followed by incubation with streptavidine Alexa Fluor® 488 conjugate or Alexa Fluor® 594 conjugate (Molecular Probes, Eugene, USA). Cover-slips were mounted onto glass slides and examined under a fluorescence microscope (Zeiss Axiovert 200). Acquisition of the immunostained cells was performed using the Image-analysis software AxioVision 4 (Carl Zeiss AG, Jena, Germany).

### Immunoblotting

Protein extracts from treated and control expanded or differentiated NPCs were prepared by centrifugation and lyses in buffer [10 mM HEPES-KOH (pH 7.9), 10 mM KCI, 1.5 mM MgCl₂, 0.1 % NP-40], supplemented with protease inhibitor cocktail (Roche Molecular Biochemicals). Protein concentration was determined using the Bradford method (Bio-Rad, Hercules, CA, USA). Cell lysates (30 µg) were mixed with sample loading buffer [125 mM Tris-HCl (pH 6.8), 4% (wt/vol) sodium dodecyl sulfate, 20% (vol/vol) glycerol, 200 mM dithiothreitol, 0.04% (wt/vol) bromphenol blue]. Proteins were resolved on a sodium dodecyl sulfate-12% polyacrylamide gel and transferred to a Hybond-ECL nitrocellulose membrane (GE Healthcare, Freiburg, Germany). Equal loading and transfer was verified after staining of membranes with Ponceau S. Membranes were blocked with 5% (wt/vol) nonfat dry milk in PBS-T (PBS, 0.1 % (vol/vol) Tween 20) for 2 hours at room temperature and subsequently incubated with desired primary antibody (diluted in PBS containing 5% non-fat milk and 0.1 % Tween 20) overnight at 4 °C with gentle agitation. The following antibodies were used: mouse monoclonal anti-proliferating cell nuclear antibody (anti-PCNA, Santa Cruz, CA, USA), mouse monoclonal anti-Bcl-2 antibody (Santa Cruz), mouse monoclonal anti-actin antibody (C4, ICN Biomedicals), rabbit polyclonal anti-tyrosine hydroxylase (anti-TH) antibody (Santa Cruz).
Membranes were washed 3 times with PBS containing 0.1% Tween20 and incubated with horseradish peroxidase-coupled secondary antibodies (Pierce, Rockford, IL, USA) for 1 h at room temperature. Chemiluminescence detection was performed by incubating the membranes with SuperSignal-Dura substrate (Pierce) followed by analyzing on a CCD cooling camera (Fuji LAS-1000plus). The chemiluminescence was quantified using AIDA, two-dimensional densitometry software (Raytest Isotopenmeb, Straubenhardt, Germany).

### Results

We have employed our novel two-step differentiation protocol compared to the control condition and compared to single step differentiation procedure employing either a depolarization or a hyperpolarization step only. Figure 1 shows that the expression of markers of mature (dopaminergic) neurons increases when culturing the cells successively in depolarization and hyperpolarization media. However, the hyperpolarization step seems to be most important.

However, electrophysiological studies reveal that the expression of neuron-specific sodium currents are most improved following the two-step protocol comprising depolarization and hyperpolarization (Figure 2). These data clearly demonstrate the benefit to neuronal differentiation using the two-step protocol.

### Expression of markers relevant for dopaminergic neurogenesis:

We used reverse transcriptase (rt) PCR to study the expression of various markers of dopaminergic neurogenesis in primary cultures from various brain regions. These data indicate that the combined epression of EN1, TH, DAT, Lmx1a, Otx2 and Neurogenin2 confirm the presence of midbrain progenitor cells (Figure 3).

### Analysis of dopaminergic marker genes during proliferation:

Figure 4 displays a variety of marker genes relevant to dopaminergic development. During proliferation relevant marker genes of immature dopaminergic neurons are continued to be expressed in respective neural stem cell cultures. Representative rt-PCR-analyses show that midbrain tissue samples (M1, M2) express a high amount of DAT, TH and EN1 in contrast to non-mesencephalic controls (B1, B2) (Panel A).

### Analysis of markers of mature dopaminergic neurons

We have also investigated markers of mature dopaminergic neurons such as dopamine receptors which are expressed on midbrain dopaminergic neurons as autoreceptors. The qualitative expression of dopamine D₂ and D₃ receptors was explored using real time (RT)-PCR and microarray analysis and quantified in expanded and differentiated cultures of NPCs Microarray analysis showed relatively low levels of expression of both D₂ and D₃ receptors in expanding mNPCs and hNPCs (data not shown). Following differentiation, expression of the autoreceptors is markedly increased in midbrain derived human NPCs (Figure 5).

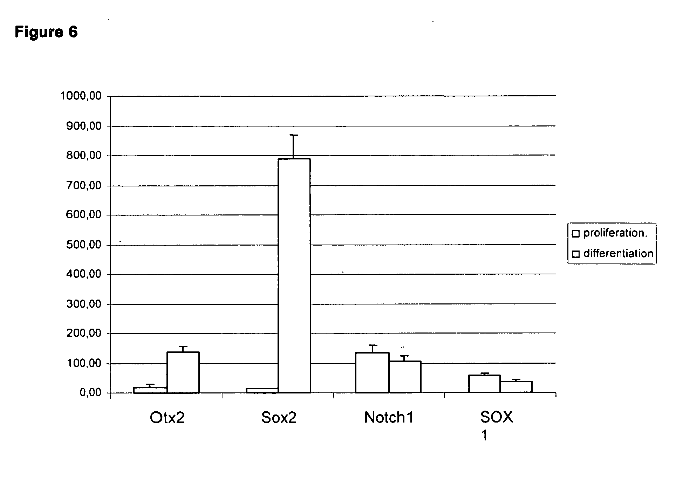

Using the above described protocols for proliferation and differentiation, we are able to maintain midbrain derived NPCs in a state where they continue to express early markers of dopaminergic neurogenesis and also increase the expression of late dopaminergic markers upon differentiation (Figure 6).

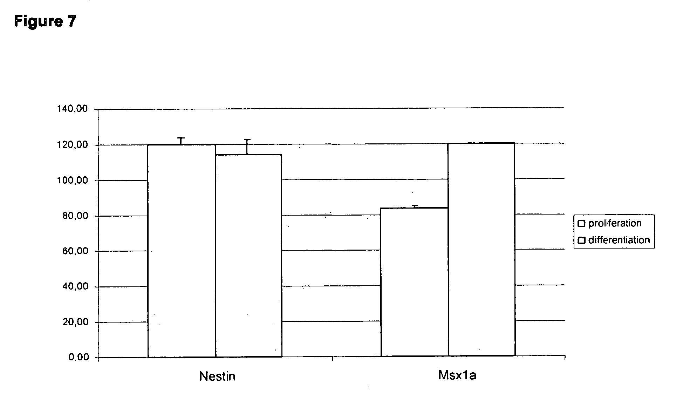

On the other hand, independent stem cell markers (Nestin and sx1) are not altered, again relative HMBS (Figure 7).

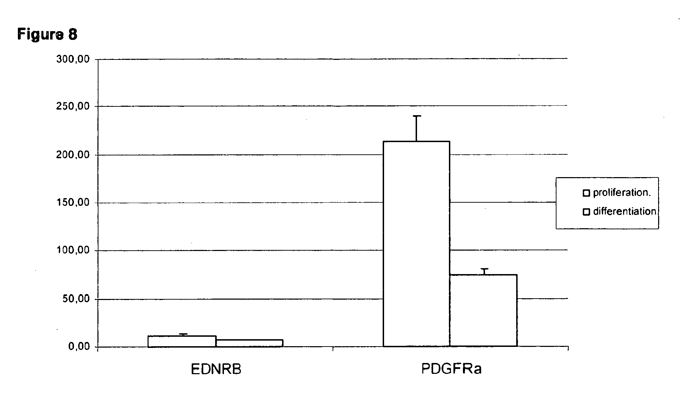

In contrary, the stem cell receptor PDGFR is markedly reduced in midbrain derived NPCs following differentiation but not the non-specific receptor EDBNR (relative to HMBS) (Figure 8).

This is the first time, where we can proof that classical markers of NPC differentiation are tremendously upregulated following differentiation of midbrain derived human NPCs (relative to HMBS)

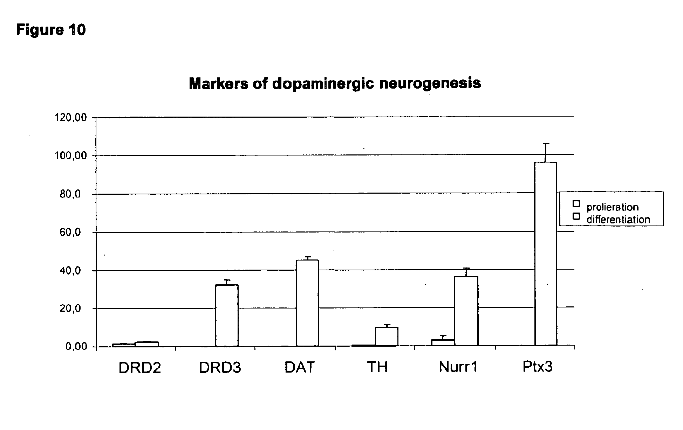

Last but not least we can convincingly show, that using the protocols indicated above, it is possible to markedly induce expression of all markers of dopaminergic development investigated (relative to HMBS) (e.g. Figure 10).

## Claims

1. A method for generating mature neuronal cells, comprising (a) expanding and/or differentiating neural precursor cells and (b) subjecting the cells obtained in step (a) to a hyperpolarization step.

2. The method according to claim 1, wherein said neural precursor cells are human neural precursor cells.

3. The method according to any one of the preceding claims, wherein the absolute value of the membrane potential of the cells during said hyperpolarization step is greater than 75 mV.

4. The method according to any one of the preceding claims, wherein said hyperpolarization step comprises culturing the cells obtained in step (a) in a medium containing Na⁺ ions and/or at least one inhibitory neurotransmitter at a concentration sufficient to cause an increase in the absolute value of the membrane potential of the cells by at least 10%.

5. The method according to claim 4, wherein said medium contains Na⁺ ions at a concentration of from 120 mM to 150 mM.

6. The method according to claim 5, wherein said medium contains 0 to 8 mM K⁺, 0 to 5 mM Mg²⁺, 0 to 5 mM Ca²⁺, 125 to 150 mM Na⁺ and 100 to 150 mM Cl⁻.

7. The method according to claim 6, wherein said medium contains 3 to 7 mM K⁺, 0.5 to 1 mM Mg²⁺, 0.5 to 1.5 mM Ca²⁺, 130 to 145 mM Na⁺ and 100 to 140 mM Cl⁻.

8. The method according to any one of claims 3 to 6, wherein the cells obtained in step (a) are cultured in said medium for 2 to 30 days.

9. The method according to any one of the preceding claims, further comprising subjecting the cells obtained in step (a) to a depolarization step prior to said hyperpolarization step.

10. The method according to claim 9, wherein the absolute value of the membrane potential of the cells during said depolarization step is less than 65 mV.

11. The method according to claim 9 or 10, wherein said depolarization step comprises culturing said neuronal cells in a depolarizing medium containing K⁺ ions and/or at least one excitatory neurotransmitter at a concentration sufficient to cause a decrease in the absolute value of the membrane potential of the cells by at least 10%.

12. The method according to claim 11, wherein said depolarizing medium contains K⁺ ions at a concentration of more than 10 mM.

13. The method according to claim 12, wherein said depolarizing medium contains 15 to 25 mM K⁺, 10 to 15 mM Mg²⁺, 3 to 7 mM Ca²⁺, 50 to 100 mM Na⁺ and 50 to 110 mM Cl⁻.

14. The method according to any one of the preceding claims, wherein said neuronal cells are cultured in said depolarizing medium for 7 to 15 days and subsequently cultured in said hyperpolarizing medium for 7 to 15 days.

15. A method according to any one of the preceding claims, wherein step (a) comprises
(i) expanding a first population of cells comprising neural precursor cells by exposing said first population of cells to at least one growth factor so as to obtain a second population of cells,
(ii) exposing said second population of cells to one or more agents inducing neuronal and/or dopaminergic differentiation so as to obtain a third population of cells comprising dopaminergic neuronal cells.

16. A method according to claim 15, wherein said one or more agents inducing neuronal and/or dopaminergic differentiation include interleukin-1.

17. A method according to claim 16, wherein said one or more agents inducing neuronal and/or dopaminergic differentiation further include forskoline, FGF-8, at least one uracil nucleotide, dexamethasone and at least one glycogen synthase kinase 3 (GSK-3) inhibitor.

18. A method according to claim 17, wherein said GSK-3 inhibitor is selected from the group consisting of kenpaullone, 6-bromoindirubin-3'-oxime (BIO) and combinations thereof.

19. The method according to claim 15, wherein step (ii) further comprises exposing the second population of cells to B27.

20. The method according to any one of claims 15 to 19, wherein the first population of cells express the markers EN1, TH, DAT, Lmx1 a, HMBS, OTX2, and Neurogenin 2 prior to the expansion step.

21. The method according to any one of claims 15 to 20, wherein the first population of cells express the markers DAT, TH, EN1, Nurr1, MAP-2 and GFAP prior to the expansion step.

22. The method according to any one of claims 15 to 21, wherein the cells express the following markers during the expansion step: wnt-1, Lmx1 a, Msx1, Ngn2 and Otx2.

23. The method according to any one of claims 15 to 22, wherein the second population of cells express the following markers: wnt-1, Lmx1 a, Msx1, Ngn2 and Otx2.

24. A mature neuronal cell obtainable by the method according to any one of the preceding claims.

25. A mature neuronal cell according to claim 24, which is capable of producing action potentials.

26. A mature neuronal cell which exhibits a voltage-dependent sodium current greater than 500 pA, preferably greater than 1000 pA, wherein said voltage-dependent sodium current is determined by patch-clamp analysis in the whole-cell voltage-clamp mode.

27. A mature neuronal cell according to any one of claims 24 to 26, which expresses the marker molecules MAP2, Tuj1, TH, DAT, DRD2, DRD3, Ptx3 and Nurr1.

28. A population of cells comprising neuronal cells, **characterized in that** at least 10% of the cells in said population of cells are mature neuronal cells according to any one of claims 24 to 27.

29. A population of cells comprising dopaminergic neurons, obtainable by the method according to any one of claims 1 to 23.

30. A population of cells comprising dopaminergic neurons, wherein
the level of expression by the cells of the marker MAP2 is at least 250
the level of expression by the cells of the marker Tuj1 is at least 500
the level of expression by the cells of the marker TH is at least 8,
the level of expression by the cells of the marker DAT is at least 10,
the level of expression by the cells of the marker DRD2 is at least 1.5,
the level of expression by the cells of the marker DRD3 is at least 10,
the level of expression by the cells of the marker Ptx3 is at least 100,
the level of expression by the cells of the marker SOX2 is at least 500,
the level of expression by the cells of the marker Nurr1 is at least 10, and
the level of expression by the cells of the marker Otx2 is at least 100,
wherein the level of expression by the cells of each of the marker proteins is indicated relative to the level of expression by the cells of the housekeeping gene hydroxymethylbilansynthase (HMBS).

31. A population of cells according to claim 30, wherein at least 10% of the cells in said population of cells are dopaminergic cells.

32. A pharmaceutical composition comprising a population of cells according to any one of claims 28 to 31.

33. The use of a population of cells according to any one of claims 28 to 31 for the manufacture of a medicament for the treatment and/or prevention of a disease affecting the nervous system.

34. The use according to claim 33, wherein said disease affecting the nervous system is a neurodegenerative disorder.

35. The use according to claim 34, wherein said neurodegenerative disorder is Parkinson's Disease.

36. The use of a population of cells for screening an active agent for the treatment and/or prevention of a disorder affecting the nervous system.


SEQUENCE LISTING

<110>  Innovalor AG

<120>  Neurons and methods for preparing them

<130>  dopaminergic neurons

<160>  76

<170>  PatentIn version 3.3

<210>  1
<211>  24
<212>  DNA
<213>  Artificial

<220>
<223>  Primer

<400>  1
gtcccgagct gtgaaggtgt ttga                                              24


<210>  2
<211>  21
<212>  DNA
<213>  Artificial

<220>
<223>  Primer

<400>  2
attgtcttcc cggtagccgc t                                                 21


<210>  3
<211>  20
<212>  DNA
<213>  Artificial

<220>
<223>  Primer

<400>  3
tccggcttcg tcgtcttctc                                                   20


<210>  4
<211>  21
<212>  DNA
<213>  Artificial

<220>
<223>  Primer

<400>  4
acaccataga accaggccac t                                                 21


<210>  5
<211>  20
<212>  DNA
<213>  Artificial

<220>
<223>  Primer

<400>  5

ctcgcagcag cctctcgtat                                                    20


<210>  6
<211>  19
<212>  DNA
<213>  Artificial

<220>
<223>  Primer

<400>  6
ccgctgctcc gtgatgtag                                                     19


<210>  7
<211>  25
<212>  DNA
<213>  Artificial

<220>
<223>  Primer

<400>  7
atcaaggcgg agcaggggga aggac                                              25


<210>  8
<211>  24
<212>  DNA
<213>  Artificial

<220>
<223>  Primer

<400>  8
tgggggtgga gaaggaggca gatt                                               24


<210>  9
<211>  19
<212>  DNA
<213>  Artificial

<220>
<223>  Primer

<400>  9
gccaaggagc ccaccaagc                                                     19


<210>  10
<211>  20
<212>  DNA
<213>  Artificial

<220>
<223>  Primer

<400>  10
atctcctcct ccagcgactc                                                    20


<210>  11
<211>  24
<212>  DNA
<213>  Artificial

<220>

<223>  Primer

<400>  11
cggacagcag tcctccatta aggt                                              24


<210>  12
<211>  24
<212>  DNA
<213>  Artificial

<220>
<223>  Primer

<400>  12
ctgaaatcgg cagtactgac agcg                                              24


<210>  13
<211>  23
<212>  DNA
<213>  Artificial

<220>
<223>  Primer

<400>  13
tccccactgc caacgtgtca gtg                                               23


<210>  14
<211>  24
<212>  DNA
<213>  Artificial

<220>
<223>  Primer

<400>  14
accctgttgc tgtagccaaa ttcg                                              24


<210>  15
<211>  20
<212>  DNA
<213>  Artificial

<220>
<223>  Primer

<400>  15
cagggaaagg aactgctgag                                                   20


<210>  16
<211>  20
<212>  DNA
<213>  Artificial

<220>
<223>  Primer

<400>  16
ctggttttgg aaccacacct                                                   20


<210>  17
<211>  20
<212>  DNA

<213>  Artificial

<220>
<223>  Primer

<400>  17
cacttcgggt atggacttgc                                                    20


<210>  18
<211>  19
<212>  DNA
<213>  Artificial

<220>
<223>  Primer

<400>  18
cgggtcttgg caaacagtg                                                     19


<210>  19
<211>  22
<212>  DNA
<213>  Artificial

<220>
<223>  Primer

<400>  19
gtactacgag accggcagca tc                                                 22


<210>  20
<211>  22
<212>  DNA
<213>  Artificial

<220>
<223>  Primer

<400>  20
cgtttcctct tctcaccatt gg                                                 22


<210>  21
<211>  20
<212>  DNA
<213>  Artificial

<220>
<223>  Primer

<400>  21
cgcatcaaga agacccgtag                                                    20


<210>  22
<211>  23
<212>  DNA
<213>  Artificial

<220>
<223>  Primer

<400>  22
gtgagtgccc agatgtagtt gtg                                                23

<210>  23
<211>  19
<212>  DNA
<213>  Artificial

<220>
<223>  Primer

<400>  23
tcggggaaac ctcaacacc                                                    19


<210>  24
<211>  19
<212>  DNA
<213>  Artificial

<220>
<223>  Primer

<400>  24
cctggcccac agcatacat                                                    19


<210>  25
<211>  20
<212>  DNA
<213>  Artificial

<220>
<223>  Primer

<400>  25
gccttccctt taaccctcac                                                   20


<210>  26
<211>  20
<212>  DNA
<213>  Artificial

<220>
<223>  Primer

<400>  26
caggagacat ggcctctagc                                                   20


<210>  27
<211>  20
<212>  DNA
<213>  Artificial

<220>
<223>  Primer

<400>  27
cagggaaagg aactgctgag                                                   20


<210>  28
<211>  20
<212>  DNA
<213>  Artificial

<220>
<223>  Primer

<400>  28

ctggttttgg aaccacacct                                                    20

<210>  29
<211>  21
<212>  DNA
<213>  Artificial

<220>
<223>  Primer

<400>  29
gtttcacctc tttgctccct g                                                   21

<210>  30
<211>  23
<212>  DNA
<213>  Artificial

<220>
<223>  Primer

<400>  30
atggcctcta gctctgttca act                                                 23

<210>  31
<211>  20
<212>  DNA
<213>  Artificial

<220>
<223>  Primer

<400>  31
cacttcgggt atggacttgc                                                     20

<210>  32
<211>  19
<212>  DNA
<213>  Artificial

<220>
<223>  Primer

<400>  32
cgggtcttgg caaacagtg                                                      19

<210>  33
<211>  20
<212>  DNA
<213>  Artificial

<220>
<223>  Primer

<400>  33
cgcatcaaga agacccgtag                                                     20

<210>  34
<211>  23
<212>  DNA
<213>  Artificial

<220>

<223>  Primer

<400>  34
gtgagtgccc agatgtagtt gtg                                                23


<210>  35
<211>  21
<212>  DNA
<213>  Artificial

<220>
<223>  Primer

<400>  35
tggctcagag gaagagtctg a                                                  21


<210>  36
<211>  21
<212>  DNA
<213>  Artificial

<220>
<223>  Primer

<400>  36
tcccccattt acatgctgtg a                                                  21


<210>  37
<211>  18
<212>  DNA
<213>  Artificial

<220>
<223>  Primer

<400>  37
gcccaggaga accccaag                                                      18


<210>  38
<211>  17
<212>  DNA
<213>  Artificial

<220>
<223>  Primer

<400>  38
cgtcttggtc ttgcggc                                                       17


<210>  39
<211>  19
<212>  DNA
<213>  Artificial

<220>
<223>  Primer

<400>  39
caggagaacc ccaagatgc                                                     19


<210>  40
<211>  17
<212>  DNA

<213>  Artificial

<220>
<223>  Primer

<400>  40
gcagccgctt agcctcg                                                    17


<210>  41
<211>  18
<212>  DNA
<213>  Artificial

<220>
<223>  Primer

<400>  41
gcaaggcaga cccgaagc                                                   18


<210>  42
<211>  22
<212>  DNA
<213>  Artificial

<220>
<223>  Primer

<400>  42
gtccaactca gccacatcaa ag                                              22


<210>  43
<211>  16
<212>  DNA
<213>  Artificial

<220>
<223>  Primer

<400>  43
ccgtccgccg agtttg                                                     16


<210>  44
<211>  19
<212>  DNA
<213>  Artificial

<220>
<223>  Primer

<400>  44
gcggtgcctg agaagattg                                                  19


<210>  45
<211>  18
<212>  DNA
<213>  Artificial

<220>
<223>  Primer

<400>  45
gcgacaacgc ctacctct                                                   18

<210> 46
<211> 18
<212> DNA
<213> Artificial

<220>
<223> Primer

<400> 46
gcacactcgt agccatcg 18


<210> 47
<211> 20
<212> DNA
<213> Artificial

<220>
<223> Primer

<400> 47
gaggcggcca gttatcagga 20


<210> 48
<211> 20
<212> DNA
<213> Artificial

<220>
<223> Primer

<400> 48
gttctcctcg ccctctagca 20


<210> 49
<211> 21
<212> DNA
<213> Artificial

<220>
<223> Primer

<400> 49
cggcaagaac tgctcactat g 21


<210> 50
<211> 18
<212> DNA
<213> Artificial

<220>
<223> Primer

<400> 50
gtgcgaggcg tcacaatg 18


<210> 51
<211> 18
<212> DNA
<213> Artificial

<220>
<223> Primer

<400> 51

ctgtgcccaa ccccattc                                                    18

<210>  52
<211>  18
<212>  DNA
<213>  Artificial

<220>
<223>  Primer

<400>  52
cgcacgccgt agtgttgg                                                    18

<210>  53
<211>  20
<212>  DNA
<213>  Artificial

<220>
<223>  Primer

<400>  53
agctcctgag cttgtccttg                                                  20

<210>  54
<211>  20
<212>  DNA
<213>  Artificial

<220>
<223>  Primer

<400>  54
tgtccacgct gtactggttc                                                  20

<210>  55
<211>  20
<212>  DNA
<213>  Artificial

<220>
<223>  Primer

<400>  55
tgcgtgccac atcaataaca                                                  20

<210>  56
<211>  25
<212>  DNA
<213>  Artificial

<220>
<223>  Primer

<400>  56
aacatccttc actcagtatt gctaa                                            25

<210>  57
<211>  18
<212>  DNA
<213>  Artificial

<220>

<223>  Primer

<400>  57
gcaagcgagt caacacca                                                    18


<210>  58
<211>  20
<212>  DNA
<213>  Artificial

<220>
<223>  Primer

<400>  58
cggtgcagag tttcatgtcc                                                  20


<210>  59
<211>  19
<212>  DNA
<213>  Artificial

<220>
<223>  Primer

<400>  59
gtctgctcca tctccaacc                                                   19


<210>  60
<211>  20
<212>  DNA
<213>  Artificial

<220>
<223>  Primer

<400>  60
ttccgtctcc tttgtttcag                                                  20


<210>  61
<211>  17
<212>  DNA
<213>  Artificial

<220>
<223>  Primer

<400>  61
ccactgagga ccccacg                                                     17


<210>  62
<211>  15
<212>  DNA
<213>  Artificial

<220>
<223>  Primer

<400>  62
tcgtccgtgg cttgc                                                       15


<210>  63
<211>  20
<212>  DNA

<213>  Artificial

<220>
<223>  Primer

<400>  63
ggcctcttct cacaagtacg                                                    20


<210>  64
<211>  21
<212>  DNA
<213>  Artificial

<220>
<223>  Primer

<400>  64
ccactctgac caaagatgaa a                                                  21


<210>  65
<211>  19
<212>  DNA
<213>  Artificial

<220>
<223>  Primer

<400>  65
tggttctggc tgtccctga                                                     19


<210>  66
<211>  25
<212>  DNA
<213>  Artificial

<220>
<223>  Primer

<400>  66
aagcagaaat agaaactgaa tagcc                                              25


<210>  67
<211>  21
<212>  DNA
<213>  Artificial

<220>
<223>  Primer

<400>  67
cctggagaag tgaaaggcaa a                                                  21


<210>  68
<211>  20
<212>  DNA
<213>  Artificial

<220>
<223>  Primer

<400>  68
ctttgacctc cctggtagcc                                                    20

<210>  69
<211>  27
<212>  DNA
<213>  Artificial

<220>
<223>  Primer

<400>  69
gcagtggtca tgccagttca ctatcag                                          27


<210>  70
<211>  29
<212>  DNA
<213>  Artificial

<220>
<223>  Primer

<400>  70
cctgttgtgt tgaaaccaaa gaggagagg                                        29


<210>  71
<211>  19
<212>  DNA
<213>  Artificial

<220>
<223>  Primer

<400>  71
gggtggagcc aaacgggtc                                                   19


<210>  72
<211>  21
<212>  DNA
<213>  Artificial

<220>
<223>  Primer

<400>  72
ggagttgctg ttgaagtcgc a                                                21


<210>  73
<211>  20
<212>  DNA
<213>  Artificial

<220>
<223>  Primer

<400>  73
cctgacagtc ctgccaaacc                                                  20


<210>  74
<211>  18
<212>  DNA
<213>  Artificial

<220>
<223>  Primer

<400>  74

gcttcctgcg gctcatcg                                                    18

<210>  75
<211>  22
<212>  DNA
<213>  Artificial

<220>
<223>  Primer

<400>  75
cctactacgc cctgtcctac tg                                               22

<210>  76
<211>  18
<212>  DNA
<213>  Artificial

<220>
<223>  Primer

<400>  76
caccacccac ggcatcac                                                    18